(19) Europäisches Patentamt
European Patent Office
Office européen des brevets

(11) **EP 2 290 611 A1**

(12) **EUROPEAN PATENT APPLICATION**

(43) Date of publication:
**02.03.2011 Bulletin 2011/09**

(51) Int Cl.:
*G06T 7/00* (2006.01)       *A61B 5/055* (2006.01)
*A61K 49/06* (2006.01)       *G01R 33/563* (2006.01)
*A61B 6/00* (2006.01)

(21) Application number: **10173819.3**

(22) Date of filing: **24.08.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **25.08.2009 JP 2009193942
30.09.2009 JP 2009226064
03.03.2010 JP 2010046361
26.03.2010 JP 2010072508**

(71) Applicant: **Fujifilm Corporation
Minato-ku
Tokyo (JP)**

(72) Inventors:
• **Masumoto, Jun
Tokyo (JP)**
• **Kobayashi, Ryoichi
Tokyo (JP)**

(74) Representative: **Klunker . Schmitt-Nilson . Hirsch
Patentanwälte
Destouchesstrasse 68
80796 München (DE)**

(54) **Medical image diagnostic apparatus and method using a liver function anagiographic image, and computer readable recording medium on which is recorded a program therefor**

(57) Performing image diagnosis in an easier and more appropriate manner by focusing on functional levels and segments of a liver. Segment function level calculation unit (33) obtains, from liver function angiographic images $V_{tn}$ obtained using a contrast agent that produces a contrast effect according to a functional level of a liver of diagnostic target, evaluations FS1, FS2, ----- representing functional levels of a plurality of liver segments $RS1_{tn}$, $RS2_{tn}$, in liver regions $RL_{tn}$, display image generation unit (34) generates an image Img based on the evaluation values, and the image Img so generated is displayed on display unit (35).

LIVER FUNCTION
(WITH RESPECT TO WHOLE LIVER) 90%
(WITH RESPECT TO SPLEEN) 90%

LIVER FUNCTION
(WITH RESPECT TO
WHOLE LIVER) 80%
(WITH RESPECT TO
SPLEEN) 70%

LIVER FUNCTION
(WITH RESPECT TO WHOLE LIVER) 120%
(WITH RESPECT TO SPLEEN) 95%

**FIG.8**

EP 2 290 611 A1

**Description**

BACKGROUND OF THE INVENTION

Field of the Invention

The present invention relates to a medical image diagnostic apparatus and method for performing image diagnosis based on a liver angiographic image obtained through the use of a contrast agent that reflects at least liver function in the image. The invention also relates to a computer readable recording medium on which is recorded a program for causing a computer to perform the method described above.

Description of the Related Art

Various types of apparatuses for performing image diagnosis using an image of a liver administered with a contrast agent have been proposed.

For example, an ultrasonic diagnostic system developed by focusing attention on the fact that, when a liver injected with a contrast agent is examined by ultrasonography, a serious liver tumor, such as primary hepatoma, liver cell carcinoma, or the like, can be detected relatively easily, in comparison with normal tissues around it or a benign tumor, by the acceptance of the contrast agent or brightening at an early stage after rapid injection of the contrast agent is known as described, for example, in U.S. Patent Application Publication No. 20090124907 (Patent Document 1) . The system searches for a pixel or voxel area, where the early stage acceptance of contrast agent or brightening occurs, from images obtained in time series after the injection of the contrast agent, discriminates the area, and highlights the positions of pixels or voxels in a parameter image representing a distribution of arrival times of the contrast agent to the liver.

As a perfusion CT application for a liver and the like, a method in which a predetermined color is allocated to each value range of perfusion parameters, such as blood flow, time to peak enhancement, and the like, and different perfusion parameters in an image are displayed in different colors is proposed as described, for example, in U.S. Patent Application Publication No. 20070016016 (Patent Document 2).

In blood flow imaging with an ultrasonography machine using a contrast agent, a method in which hepatic artery and portal vein are displayed discriminately by displaying a plurality of blood flows having different arrival times or levels of a contrast agent with different colors is proposed as described, for example, in U.S. Patent No. 6,582,370 (Patent Document 3).

An apparatus that performs segmentation through binarization or region growing method on an X-ray CT image of a liver administered with a contrast agent to extract portal veins, liver parenchymas, and a tumor area, then identifies as to which portal vein control area the detected tumor belongs based on the positions of the core lines of extracted portal veins and blood vessel diameters to identify the portal vein supplying nourishment to the tumor, identifies the liver parenchyma controlled by the identified portal parenchyma as a resection area, and displays the identified area in a different hue from other areas is also proposed as described, for example, in Japanese Unexamined Patent Publication No. 2003-033349 (Patent Document 4).

As for the apparatus for reconstructing and displaying two-dimensional cross-sectional images from three-dimensional data, an apparatus that displays three orthogonal sectional images, namely axial, sagittal, and coronal images, is well known as described, for example, in U.S. Patent No. 7,315,638 (Patent Document 5).

An apparatus used for performing comparative reading of a plurality of series of medical images, in which sectional images viewed from the same direction and at the same sectional position are generated based on medical image data of each series and displayed is known as described, for example in U.S. Patent Application Publication No. 20070242069 (Patent Document 6).

Another apparatus that serially radiographs the same region, such as a heart or the like, in one radiography operation to obtain a plurality of sets of volume data imaged at different times, then generates a representative image of each set of volume data and thumbnail displays each representative image, and displays, on each thumbnail displayed representing image, a marker indicating the imaging time of the volume data in a superimposing manner is also known as described, for example, in Japanese Unexamined Patent Publication No. 2009-148422 (Patent Document 7).

In the mean time, a contrast agent that includes gadoxetate sodium (Gd-EOB-DTPA) as an active ingredient is developed (hereinafter, referred to as "EOB contrast agent") as a liver specific MRI contrast agent. The gadoxetate sodium has a structure having a basic skeleton similar to that of gadobenate dimeglumine (Gd-DTPA), which is a conventional nonspecific extracellular fluid contrast agent, with benzene ring and lipophilic ethoxybenzyl group (EOB) added to the skeleton. This increases the cell membrane permeability due to increased lipophilicity, whereby the gadoxetate sodium is selectively absorbed in normally functioning liver cells. Consequently, due to a contrast between a normal liver cell (having a high signal value) and an impaired or lost liver cell (having a low signal value) in an image, it is expected that the use of gadoxetate sodium allows detection of tumors (e.g., cyst, metastatic liver cancer, most of liver cell carcinomas) viewed from the aspect of liver cell function, in particular, detection of small tumors and distinction between benign and malignant liver tumors which have not been realized by previous contrast agents as described, for example, in an online literature by H. Nakagawa, Gifu Association of radiological Technologists, Seinoh Branch, Retrieved on 24th of July, 2008, <URL: http://plaza.umin.ac.jp /~GifuART/sibu/seino/2008_seino_HP/pdf/dailkai/syouroku_nakagaw a.pdf> (Non-patent Document 1), a literature by S. Saito, Journal of Japanese Society of Radiological Technology, Vol.51, pp. 30-33, 2008 (Non-patent Document 2), an online literature by Y. Kato, Nikkei Medical Online, Retrieved on 10th of July, 2009, <URL: http://medical.nikkeibp.co.jp/leaf/all/search/cancer/news/20080 2/505562.html> (Non-patent Document 3), and H-K. Ryeom et al., "Quantitative Evaluation of Liver Function with MRI Using Gd-EOB-DTPA", Korean Journal of Radiology, Vol. 5, No. 4, pp. 231-239, 2004 (Non-patent Document 4).

In a contrast examination of a liver using an EOB contrast agent, contrast effects appear in a different region according to the elapsed time from the administration of the contrast agent. More specifically, in an arterial phase radiographed at a time 20 to 35 seconds after the administration of the contrast agent, an area of abundant blood vessels in the liver is strongly enhanced by the EOB contrast agent flowed into the liver from the hepatic artery. Then, in an equilibrium phase and in a hepatocyte phase which are phases 3 minutes and 20 minutes after the administration of the contrast agent respectively, normal cells are strongly enhanced because the EOB contrast agent is absorbed in the normal cells while the concentration of the EOB contrast agent in the area of abundant blood vessels initially enhanced strongly is reduced.

Therefore, it is regarded as important, in image diagnosis of liver using EOB contrast agent, to make a comprehensive judgment based on images obtained in a plurality of time phases after administration of the contrast agent. For example, if a plethoric liver tumor exists in a liver parenchyma, that portion is strongly enhanced in the arterial phase and appears as a high signal area in the image. In contrast, the concentration of the contrast agent in the plethoric liver tumor is reduced in the equilibrium phase or hepatocyte phase and appears as a low signal area in the image since the tumor portion is not a normal portion of the liver parenchyma and the EOB contrast agent is not absorbed in the portion. That is, based on the fact that a plethoric liver tumor is imaged first as a high signal area by reflecting blood flow in the arterial phase and as a low signal area by reflecting abnormality of liver function in the equilibrium phase and hepatocyte phase, a time series analysis of variation in signal value in each time phase allows highly accurate detection/distinction of a plethoric liver tumor as described, for example, in Non-patent Document 2 and Non-patent Document 3.

The EOB contrast agent is distributed in blood vessels and intercellular gaps after injected in a vein, and allows a contrast image of liver cells to be taken from 20 minutes after the injection. The signal enhancement effect continues at least 2 hours and the agent is eventually excreted in urine or bile. Therefore, the use of the EOB contrast agent allows not only the diagnosis based on blood flow evaluation using hemodynamics images from right after the injection of the contrast agent to the excretion, but also the evaluation of liver cell function in the hepatocyte phase as described, for example, in Non-patent Documents 1 through 4.

Then a method of evaluating liver function by obtaining, through the use a dynamic MRI image obtained by using the EOB contrast agent, time series variations (time intensity curves (TIC)) of contrast agent concentrations in the abdominal aorta and liver parenchyma, and analyzing the time intensity curves by a deconvolution method is proposed, for example, in Non-patent Document 4.

Diagnosis or surgery of a liver is generally performed on a segment-by-segment basis classified according to control areas of hepatic artery, portal vein, and hepatic vein, and as a specific example of such classification method, Couinaud's subsegmental classification method or the like is known. Therefore, in the case in which a target region for resection surgery is determined, the determination should be made on a segment-by-segment basis. Further, in such a case, it is required to determine a resection target by focusing on a functional level of each segment, i.e., how well each segment of the liver is functioning.

In contrast, the technology described in Patent Document 1 uses a contrast agent (micro bubble) absorbed in liver cells, but intends to the detection of a liver tumor, not the evaluation of liver cell function. The technologies described in Patent Documents 1 to 3 do not pay attention to liver segments. In the mean time, the technology described in Patent Document 4 performs liver segment identification but does not intend to the evaluation of liver function. Further, the technology described in Non-patent Document 4 performs the evaluation of liver cell function using an EOB contrast agent but does not pay attention to liver segments. These conventional technologies, therefore, can not be said to appropriately meet the needs in image diagnosis of liver described above.

In order to effectively perform image diagnosis for a liver using an EOB contrast agent, it is necessary to accurately understand a time series variation in signal value of a region of interest in a plurality of imaging time phases.

In contrast, the parameter images described in Patent Documents 1 and 2 are images compressed in a time axis direction. It is, therefore, difficult to understand a time series variation of signal value of a region of interest in detail. Further, it is unrealistic to make a judgment based only on a parameter image in an actual image diagnosis site, and the judgment is made by returning to the original image. Thus, it is demanded that a plurality of original images be displayed in a manner appropriate for improving diagnostic efficiency.

Now, a discussion will be made for the employment of the conventional technologies of medical image display described above for that purpose. For example, a modification may be made to the image display apparatus described in Patent Document 5 such that the entire screen is divided into a number of small areas corresponding to the number of imaging phases desired to be displayed and three orthogonal sectional images in one imaging phase are displayed in each small area, thereby displaying three orthogonal sectional images for a plurality of phases side-by-side on the screen. In this case, however, the size of each sectional image of each imaging phase becomes too small to understand the time series variation in signal value of a region of interest in detail due to the constraint of screen size. In addition, the displayed sectional image represents the whole test body in that section so that it is necessary to identify the region of interest in each sectional image. Therefore, the apparatus discussed above can not be said to have a satisfactory function from the viewpoint of efficiently understanding a time series variation in signal value of a region of interest.

Further, in the medical image display apparatus described in Patent Document 6, if it is assumed that each medical image of each series corresponds to each medical image of each imaging phase after administration of an EOB contrast agent, the apparatus may display sectional images of a plurality of imaging phases in parallel. But, when the number of series, i.e., the number of imaging phases is increased, the size of each sectional image becomes small since the displayed sectional image represents the whole test body in that section. Therefore, it is difficult to understand a time series variation in signal value of a region of interest in detail, as in the case described above. Further, it also poses a problem in terms of effective identification of a region of interest.

Still further, the apparatus described in Patent Document 7 may display a list of medical images of respective imaging phases, but the representative image displayed at each imaging time does not always include a region of interest, and the representative image is reduced. Therefore, it is difficult understand a time series variation in signal value of a region of interest in detail.

The present invention has been developed in view of the circumstances described above, and it is a primary object of the present invention to provide a medical image diagnostic apparatus and method that allows more valuable and practical image diagnosis using a liver angiographic image obtained through the use of a contrast agent that reflects liver function in an image. It is another primary object of the present invention to provide a computer readable recording medium on which is recorded a program for causing a computer to perform the method described above.

More specifically, it is a secondary object of the present invention to allow easier and more appropriate image diagnosis by focusing on functional levels and segments of a liver.

It is another secondary object of the present invention to allow understanding of a time series variation in signal value of a region of interest in each time phase in detail and efficiently based on liver angiographic images obtained in a plurality of time phases in a contrast examination using a contrast agent that reflects liver blood flow and liver function in an image.

SUMMARY OF THE INVENTION

A first medical image diagnostic apparatus of the present invention is an apparatus, including:

a segment functional level information obtaining means for obtaining, from a liver function angiographic image which is obtained after a predetermined time from administration to a test body of a contrast agent that produces a contrast effect according to a functional level of a liver and which represents a functional level of the liver of the test body, segment functional level information representing a functional level of at least one segment of the liver of the test body; and

a segment functional level presentation means for presenting the obtained segment functional level information.

A first medical image diagnostic method of the present invention is a method, including the steps of:

obtaining, from a liver function angiographic image which is obtained after a predetermined time from administration to a test body of a contrast agent that produces a contrast effect according to a functional level of a liver and which represents a functional level of the liver of the test body, segment functional level information representing a functional level of at least one segment of the liver of the test body; and

presenting the obtained segment functional level information.

A first computer readable recording medium of the present invention is a medium on which is recorded a medical image diagnostic program for causing a computer to perform the first method described above.

The term "a functional level of a liver" as used herein refers to a normally functioning level of the liver. The "contrast agent that produces a contrast effect according to a functional level of a liver" may be a contrast agent that produces a contrast effect that directly represents the functional level of the liver or a contrast agent that produces a contrast effect that indirectly represents the functional level of the liver. The former contrast agent may be an agent absorbed by liver cells such that the higher (or lower) the functional level of the liver, the more amount is absorbed, a selective contrast agent which is absorbed only when the functional level of the liver is normal, or a contrast agent which is absorbed by liver cells by an amount within a certain range when the liver function is normal, while if the liver function is abnormal, by an amount more than or less than the certain amount range. A liver function angiographic image obtained with a contrast agent that produces such contrast effect reflects a normality level of a liver, in particular, of a liver cell as a magnitude of pixel value. Specific examples of such contrast agents include an EOB contrast agent that includes gadoxetate sodium (Gd-EOB-DTPA) as an active ingredient and is used in MRI, superparamagnetic iron oxide (SPIO contrast agent), asialo scinti (99mTc-GSA) used in SPECT, and the like. The latter contrast agent may include, for example, a contrast agent whose primary purpose is to analyze blood flow of a liver, in which the use of a parameter representing a level of blood pooling allows indirect evaluation of the liver function. An iodine based contrast agent used in CT is one of such type of contrast agents.

The term "at least one segment of the liver" may be a plurality of segments, in which case, it is preferable that the segment functional level information is presented with respect to each of the plurality of segments.

As for the presentation method of the segment functional level information, it is preferable that the segment functional level information is presented in a manner that allows the difference in functional level with respect to each of the segments is visually identifiable. For example, when segment functional level information of a plurality of segments is presented, it is possible to present each segment in a different color according to the functional level of each segment. Further, the segment functional level information may be presented with character information, an icon, or the like.

The modality for obtaining the liver function angiographic image is selected according to the contrast agent used. For example, when an EOB contrast agent or a SPIO contrast agent is used, the liver function angiographic image is an image obtained by MRI or the like, and images representing a time series variation in a three-dimensional space obtained by dynamic MRI or the like are particularly preferable. In the case of asialo scinti, the liver function angiographic image is an image obtained by SPECT.

Here, the liver function angiographic image may sometimes does not clearly represents an anatomical structure of a liver, although it appropriately represents liver function. In such a case, an arrangement may be adopted in which a morphological image that appropriately represents an anatomical structure of a liver is obtained separately, then each segment of the liver in the morphological image is identified, and each segment in the liver function angiographic image corresponding to each segment identified from the morphological image is identified as each segment of the liver. Here, "each segment in the liver function angiographic image corresponding to each segment identified from the morphological image" may be identified by aligning the liver positions between the morphological image and liver function angiographic image and using the alignment results. A specific example of the morphological image is an image obtained by CT.

The "segment" may be a three-dimensional region, and it is preferable that the segment functional level information is displayed three-dimensionally. Specific examples of "three-dimensional display" may include a method in which a pseudo three-dimensional image is generated by volume rendering or the like and displayed, and a method in which sectional images viewed from a plurality of view points (e.g., sectional images of three orthogonal sections) are generated and displayed. The segment functional level may a functional level that includes a time series variation in functional level of the three-dimensional segment.

Further, the liver function angiographic image may be an image that further includes image information of a reference region other than the liver, and the functional level of the segment maybe obtained as a relative relationship with respect to a functional level of the reference region. A spleen region may be cited as a specific example of the reference region.

A second medical image diagnostic apparatus of the present invention is an apparatus, including:

a region of interest setting means for setting, with respect to each of images of two or more time phases of interest among liver angiographic images obtained in a plurality of time phases in a contrast examination using a contrast agent that reflects liver blood flow and liver function in an image, a region of interest in the liver of test body positionally corresponding to each other between each of the time phases of interest;
a local image generation means for generating a local image representing the region of interest with respect to each of the time phases of interest based on each of the liver angiographic images of the time phases of interest; and
a display control means for causing the generated local image with respect to each of the time phases of interest to be displayed in a manner that allows comparative reading.

A second medical image diagnostic method of the present invention is a method, including the steps of:

setting, with respect to each of images of two or more time phases of interest among liver angiographic images obtained in a plurality of time phases in a contrast examination using a contrast agent that reflects liver blood flow and liver function in an image, a region of interest in the liver of test body positionally corresponding to each other between each of the time phases of interest;

generating a local image representing the region of interest with respect to each of the time phases of interest based on each of the liver angiographic images of the time phases of interest; and

a display control means for causing the generated local image with respect to each of the time phases of interest to be displayed in a manner that allows comparative reading.

A second computer readable recording medium of the present invention is a medium on which is recorded a medical image diagnostic program for causing a computer to perform the second method described above.

In the present invention, the liver angiographic image is an image obtained in a contrast examination using a contrast agent that reflects liver blood flow and liver function in the image, in which the liver blood flow and liver function are reflected as magnitudes of pixel values.

Preferably, the contrast agent used in the contrast examination is a contrast agent that reflects the liver blood flow and liver function in the image at different times. A contrast agent that includes gadoxetate sodium (Gd-EOB-DTPA) as an active ingredient is cited as a specific example.

Further, in the contrast examination, each of a plurality of time phases at which the liver angiographic image is obtained is used as a time phase of interest or two or more time phases of interest may be selected from the plurality of time phases. A specific selection method conceivable is a method in which time phases of interest are selected according to the elapsed time from administration of the contrast agent. For example, it is possible to select time phases of interest at a predetermined time interval after administration of the contrast agent or predetermined times (e.g., 20 sec, 70 sec, 3 min, 20 min) after administration of the contrast agent are selected as the time phases of interest. Here, it is preferable that an arterial phase, which is a time phase when the contrast agent is flowing into blood vessels in the liver from the hepatic artery, and a hepatocyte phase, which is a time phase when the contrast agent is absorbed by normal liver cells, are included in the time phases of interest.

The term "setting a region of interest in the liver positionally corresponding to each other between each of the time phases of interest" refers not to set a separate region of interest in the liver angiographic image in each time phase of interest but to set the same region of interest in the liver angiographic image in each time phase of interest. Consequently, the region of interest so set positionally corresponds to each other between each time phase of interest. More specifically, it is possible to set a region of interest in the liver angiographic image in a certain time phase of interest by a manual operation of the user, automatic setting based on image analysis, or a combination thereof, and a region of the liver angiographic image in another time phase of interest having the same coordinate values as those of the previously set region of interest is set as the region of interest. In this case, it is preferable to take into account the displacement of the region of interest between time phases due to body movement, respiration, and the like of the test body and to set the size of the local image larger than a maximum possible displacement between time phases of interest. Alternatively, it is possible to set a region of interest in the liver angiographic image in a certain time phase of interest by taking into account such displacement, and a region of interest is set in each liver angiographic image of other time phases of interest corresponding to the previously set region of interest based on a content feature of the liver angiographic image in each time phase of interest. More specifically, it is conceivable that correspondence relationship of coordinate values of corresponding positions between time phases is obtained in advance by performing positional alignment on the liver angiographic image in each time phase of interest, and a region of interest is set in the liver angiographic image in another time phase of interest by transforming the coordinate values of the previously set region of interest based on the correspondence relationship. Further, the positional alignment may be performed manually.

The local images representing the region of interest in the respective time phases are images for observing a time series variation of the region of interest in detail. Therefore, these images are preferable to be those on which an image reduction operation or a pixel skipping operation is not performed. Further, if the liver angiographic image in each time phase is a three-dimensional image and the region of interest is a three-dimensional region, a local image representing the region of interest viewed from each of a plurality of directions may be generated with respect to each time phase. More specifically, it is conceivable to generate sectional images of three orthogonal sections of axial, sagittal, and coronal sections passing through the region of interest with respect to each time phase.

When displaying the local image with respect to each of the time phases of interest, specific examples of the manner that allows comparative reading include a manner in which the local images of the respective time phases of interest are displayed side-by-side in one screen and a manner in which the local images of the respective time phases of interest are sequentially switched and displayed in the order of time.

Further, in the present invention, an arrangement may be adopted in which a whole image representing an area that includes at least the whole liver of the test body and the region of interest is generated based on liver angiographic images obtained in a plurality of time phases, and the local images and the generated whole image are displayed at the same time.

If the liver angiographic image in each time phase is a three-dimensional image and the region of interest is a three-dimensional region, a whole image viewed from each of the plurality of directions may be generated. Here, it is preferable that the local images and the whole images are images viewed from the same direction. More specifically, it is conceivable to generate sectional images of three orthogonal sections of axial, sagittal, and coronal sections passing through the region of interest based on the liver angiographic image of a creation time phase of interest. Here, an arrangement may be adopted in which the time phase of interest for the liver angiographic image serving as the source for generating the whole image is selectable by a user operation. The whole image may be a pseudo three-dimensional image obtained by performing volume rendering on the three-dimensional liver angiographic image in a certain time phase. Further, a functional image representing liver function may be used as the whole image or an image formed by superimposing a morphological image, which is like the aforementioned sectional image, and the functional image on top of each other may be used as the whole image. The liver functional image may be generated, for example, by extracting a liver region from a liver angiographic image, calculating, with respect to at least some of a plurality of small areas obtained by dividing the extracted liver region, a liver function evaluation value representing a functional level of each small area, and based on the liver angiographic image and the calculated liver function evaluation values. Each small area obtained by dividing the liver region may be one pixel (voxel) area, an area of several pixels, or an area of a size corresponding to a size when the liver region is divided into several segments like Couinaud's segments.

Further, an arrangement may be adopted in which the whole image is an image in which the region of interest is identifiable, as in the sectional images of three orthogonal sections, and a marker representing the region of interest is superimposed on the whole image. Still further, an arrangement may be adopted in which the marker is movable by the user using an input means, the position of the marker after moved is detected, a region of interest is set in a liver angiographic image serving as the source for generating the whole image based on the detected position of the marker, and a corresponding region of interest is set in the liver angiographic image in each time phase of interest other than the liver angiographic image in which the region of interest is set. Still further, a marker may be displayed superimposed on the region of interest in a local image.

Still further, an arrangement may be adopted in which a liver function evaluation value of the region of interest is calculated with respect to each time phase of interest and a time series variation in the liver function evaluation value of the region of interest between time phases is visualized in the form of a graph or the like.

According to the present invention, more practical image diagnosis can be made using a liver angiographic image obtained by using a contrast agent that reflects liver function in an image.

More specifically, according to the first embodiment of the present invention, segment functional level information representing a functional level of at least one segment of a liver of diagnostic target is obtained from a liver function angiographic image obtained with a contrast agent that produces a contrast effect according to a functional level of a liver of diagnostic target and presented. This allows easier and more appropriate image diagnosis to be made using the functional level and segments of the liver. For example, when a target region of a liver for resection surgery is determined through image diagnosis of the liver, the functional level of the liver may be confirmed on a segment-by-segment basis, so that a determination may be made easily and appropriately as to which segment is to be a resection target.

According to the second embodiment of the present invention, with respect to each of liver angiographic images of two or more time phases of interest obtained in a contrast examination using a contrast agent that reflects liver blood flow and liver function in an image, a region of interest positionally corresponding to each other between each of the time phases of interest is set, a local image representing the region of interest is generated with respect to each of the time phases of interest based on each of the liver angiographic images of the time phases of interest, and the generated local image with respect to each of the time phases of interest is displayed in a manner that allows comparative reading. This allows comparative reading of the displayed local images of the respective time phases of interest to be made easily, and a time series variation in signal value of the region of interest in each time phase of interest may be understood in detail and efficiently.

That is, the second embodiment of the present invention may achieve a time series variation in signal value of the region of interest among a plurality of time phases, which has not been achieved by conventional technologies of displaying parameter images as described in Patent Documents 1 and 2, by performing image reading on the displayed local image of each time phase of interest. Further, the present invention may display only a region of interest, which is a portion of a whole image, so that, instead of performing image reading on a region of interest of a reduced whole image as in the conventional technologies described in Patent Documents 3 to 5, image reading may be performed on the local image which is at least not so reduced as the whole image, if the screen size used is the same as that of conventional technologies, and, therefore, the region of interest in each time phase of interest may be observed in detail. Further, since only the region of interest is displayed in the second embodiment of the present invention, it is not necessary to identify a region of interest in the whole image, as required in the conventional technologies described in Patent Documents 5 to 7, whereby image reading efficiency is improved.

As described above, the second embodiment meets the demand of accurately understanding a time series variation in signal value of a region of interest in a plurality of time phases in a contrast examination using a contrast agent that reflects liver blood flow and liver function in an image and is extremely useful for image diagnosis of a liver using such a contrast agent.

BRIEF DESCRIPTION OF THE DRAWINGS

Figure 1 is a schematic diagram of a medical image diagnostic system using a liver function angiographic image in each embodiment of the present invention.

Figure 2 is a block diagram, schematically illustrating a configuration and a process flow for realizing a medical image diagnostic function using a liver function angiographic image in a first embodiment of the present invention.

Figure 3 is a graph illustrating a time series variation (a time intensity curve (TIC)) of pixel values of a liver function angiographic image.

Figure 4 is a schematic view of an example configuration of a display screen in an embodiment of the present invention.

Figure 5 illustrates an example user interface for extracting a liver region.

Figure 6 illustrates an example user interface for identifying a liver segment.

Figure 7 illustrates an example volume rendering image in which each function evaluation value with respect to each liver segment is displayed in a different color.

Figure 8 illustrates another example of volume rendering image in which each function evaluation value with respect to each liver segment is displayed in a different color.

Figure 9 is a schematic block diagram, illustrating a configuration and a process flow for realizing a medical image diagnostic function using a liver function angiographic image in a second embodiment of the present invention.

Figure 10 is a schematic block diagram, illustrating a configuration and a process flow for realizing a medical image diagnostic function using a liver angiographic image in a third embodiment of the present invention.

Figure 11 is a schematic view of an example configuration of a display screen in the third embodiment of the present invention.

Figure 12A illustrates an example user interface for selecting a display target time phase of a whole image.

Figure 12B illustrates another example of user interface for selecting a display target time phase of a whole image.

Figure 13 is a chart illustrating operation transitions of the medical image diagnostic system in the third embodiment of the present invention.

Figure 14 illustrates an example image display in the third embodiment of the present invention.

Figure 15 illustrates an example case in which a marker is superimposed on a region of interest of a local image in an axial image display area.

Figure 16 illustrates another example of image display in the third embodiment of the present invention.

Figure 17 is a block diagram, schematically illustrating a configuration and a process flow of a modification of the third embodiment of the present invention.

Figure 18 illustrates an example image display in the modification of the third embodiment of the present invention.

Figure 19 is a block diagram, illustrating liver region extraction unit in detail.

Figure 20 illustrates an example of a reference point and an adjacent area of a liver region.

Figure 21 illustrates a method of extracting a target region by the region extraction unit shown in Figure 19.

Figure 22 illustrates a method of setting values of s-link and t-link based on the positions of seed point and reference point.

Figure 23 illustrates another method of extracting a target region by the region extraction unit shown in Figure 19.

DESCRIPTION OF THE PREFERRED EMBODIMENTS

Hereinafter, a medical image diagnostic system using a liver function angiographic image according to an embodiment of the present invention will be described with reference to the accompanying drawings.

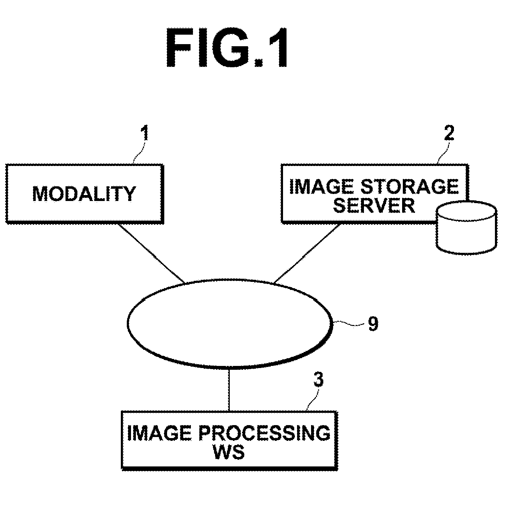

Figure 1 is a hardware configuration diagram of the medical image diagnostic system, illustrating an overview thereof. As shown in the drawing, the system includes modality 1, image storage server 2, and image processing workstation 3 communicatably linked to each other via network 9.

In the first embodiment of the invention, modality 1 includes an MRI system. The MRI system is capable of performing dynamic imaging in which three-dimensional medical images (voxel data) $V_{t0}$, $V_{t1}$, -----, $V_{t6}$ (hereinafter, collectively referred to as thee-dimensional dynamic images $V_{tn}$) of an abdomen (including liver) are obtained before administering a contrast agent that includes gadoxetate sodium (Gd-EOB-DTPA) as an active ingredient (EOB contrast agent) and, for example, 20 sec, 1 min, 2 min, 5 min, 10 min, and 20 min after administration of the EOB contrast agent. Here, as described above, the EOB contrast agent has a property to be selectively absorbed in normal liver cells, so that thee-dimensional dynamic images $V_{tn}$ obtained by such imaging correspond to liver function angiographic images.

Image storage server 2 is a computer for storing image data of thee-dimensional dynamic images $V_{tn}$ obtained by modality 1 and of medical images generated through image processing in image processing workstation 3 in a database and managing, and includes a large capacity external memory unit and database management software (e.g.,

object relational database (ORDB)).

Image processing workstation 3 is a computer that performs, in response to a request from a radiological reader, image processing (including image analysis) on thee-dimensional dynamic images $V_{tn}$ obtained from modality 1 or image storage server 2 and displays a generated image. It includes an input device, such as a keyboard, a pointing device, or the like, for receiving a request from a radiological reader, a main storage unit with a capacity capable of storing at least a portion of the obtained thee-dimensional dynamic images $V_{tn}$, and a display for displaying a generated image. The process of medical image diagnosis of the present invention is installed in the image processing workstation, and the process is realized by executing a program installed from a recording medium, such as a CD-ROM or the like. Alternatively, the program may be a program installed after being downloaded from a storage unit of a server connected via a network, such as Internet or the like.

The storage format of image data and communication between each component of the system are based on a protocol, such as DICOM (Digital Imaging and Communication in Medicine) or the like.

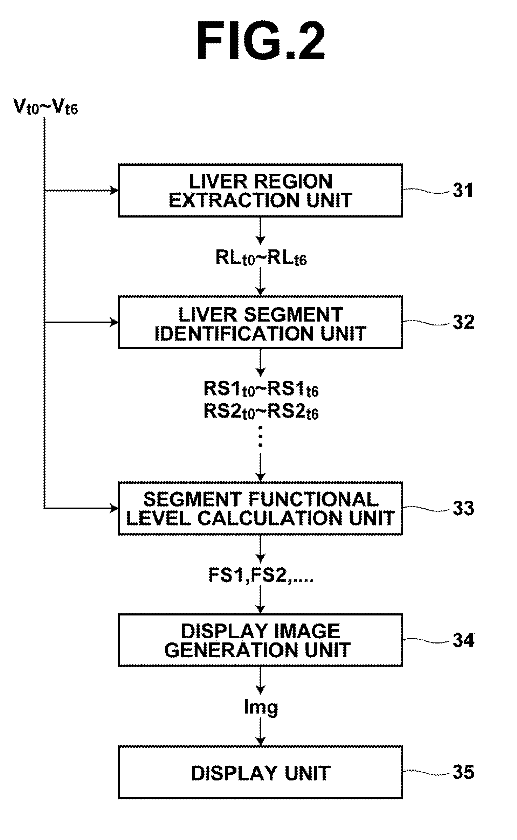

Figure 2 is a block diagram, illustrating a portion of the function of image processing workstation 3 relevant to the process of medical image diagnosis according to the first embodiment of the present invention. As shown in the drawing, the process of medical image diagnosis according to the first embodiment of the present invention is realized by liver region extraction unit 31, liver segment identification unit 32, segment functional level calculation unit 33, display image generation unit 34, and display unit 35. Each unit will now be described in detail.

Liver region extraction unit 31 extracts liver regions $RL_{tn}$ from thee-dimensional dynamic images $V_{tn}$ using, for example, a method proposed by the present applicant in Japanese Patent Application No. 2008-050615.

More specifically, with respect to a three-dimensional image in a creation phase (e.g., a three-dimensional image $V_{t6}$ in hepatocyte phase having a large contrast between a liver region and an adjacent area), an arbitrary point in the liver region is set by the user (hereinafter, referred to as "user set point") by operating the pointing device or the like and an angulated portion of a contour of the liver region is detected as a reference point using a discriminator obtained through machine learning. Then, with respect to each point (voxel) in a three-dimensional area (hereinafter, referred to "processing target area") of a size sufficient to include the liver region centered on the user set point, an evaluation value that indicates whether or not each point is a point on the contour of the liver region is calculated using the discriminator obtained through machine learning to determine each point on a circumference of the processing target area and a liver region $RL_{t6}$ is extracted from the three-dimensional image $V_{t6}$ by further applying a graph-cut method using an evaluation value of each point within the processing target area (for more detailed explanation, refer to the last section herein under "Supplementary Explanation").

For the three-dimensional images in other time phases (e.g., $V_{t0}$, -----, $V_{t5}$), liver regions $RL_{t0}$, -----, $RL_{t5}$ may be extracted from the respective three-dimensional images using a method identical to that described above, or an area of each of the three-dimensional images with coordinate values corresponding to those of the liver region $RL_{t6}$ extracted from the three-dimensional image $V_{t6}$ may be extracted as each of the liver regions $RL_{t0}$, -----, $RL_{t5}$.

For extracting a liver region, other known methods, such as the method described in U.S. Patent No. 7, 046, 833, may also be used.

Liver segment identification unit 32 identifies a plurality of liver segments $RS1_{tn}$, $RS2_{tn}$, ------ in each of liver regions $RL_{tn}$ in each time phase. More specifically, when a planar or curved surface of boundary of a plurality of liver segments in a liver region (e.g., liver region $RL_{t6}$ in the hepatocyte phase) is specified by the user by operating the pointing device or the like, a plurality of liver segments (e.g., $RS1_{t6}$, $RS2_{t6}$, ------) in the liver region is identified. Each of the plurality of identified liver segments is a three-dimensional area. For liver regions of other time phases (e.g., liver regions $RL_{t0}$, -----, $RL_{t5}$), liver segments $RSI_{t0}$, ----- $RS5_{t0}$, $RS1_{t1}$, ----- $RS5_{t1}$, ------, $RS1_{t5}$, ----- $RS5_{t5}$ in each of time phases may be identified using the same method as described above, or an area with coordinate values corresponding to those of each of liver segments $RS1_{t6}$, ----- $RS5_{t6}$ identified in the liver region $RL_{t6}$ may be identified as each of liver segments in each time phase.

For identifying a liver segment, other known methods, including the following may also be used. That is, a method that extracts blood vessels in a liver region and determines that each point in an area of the liver region other than the blood vessels (liver parenchyma and the like) is controlled by a blood vessel located nearest to the point using a Voronoi diagram, i.e., identifies to which blood vessel control area each area of the liver region other than the blood vessels belongs, thereby identifying a control area of each blood vessel as a liver segment as described, for example, in Patent Document 4 described above and R. Beichel et al., "Liver Segment Approximation in CT Data for Surgical Resection Planning", Medical Imaging 2004: Image Processing, Proceedings of the SPIE, Vol. 5370, pp. 1435-1446, 2004.

Segment functional level calculation unit 33 calculates evaluation values FS1, FS2, ----- representing liver functional levels for liver segments $RS1_{tn}$, $RS2_{tn}$, ----- respectively based on the three-dimensional dynamic images $V_{tn}$. That is, segment functional level calculation unit 33 calculates an evaluation value with respect to each liver segment, like calculating the evaluation value FS1 based on image information of liver segments $RS1_{t0}$, $RS1_{t1}$, -----, $RS1_{t6}$, the evaluation value FS2 based on image information of liver segments $RS2_{t0}$, $RS2_{t1}$, -----, $RS2_{t6}$, and so on.

Here, specific examples of the evaluation value with respect to each liver segment may include the following.

(1) Average Pixel Value

An average value obtained by averaging a pixel value of each pixel with respect to each liver segment in each time phase

(2) Maximum Average Pixel Value

A maximum average value of those obtained by averaging a pixel value of each pixel with respect to each liver segment and each time phase

(3) Minimum Average Pixel Value

A minimum average value of those obtained by averaging a pixel value of each pixel with respect to each liver segment and each time phase

(4) Under Curve Area in Time Series Variation in Average Pixel Value

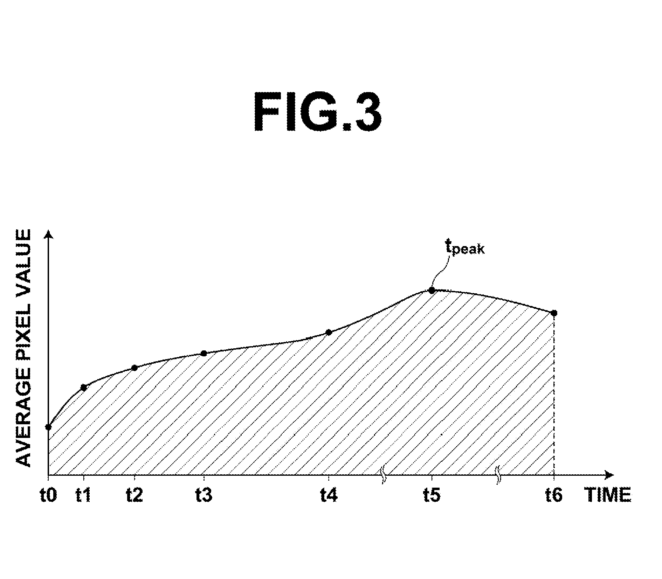

An under curve area obtained by averaging a pixel value of each pixel with respect to each liver segment and each time phase, and representing a time series variation in the average pixel value with respect to each liver segment in a graph with the horizontal axis indicating each time phase (time) and the vertical axis indicating the average pixel values, as shown in Figure 3 (shaded portion in Figure 3)

(5) Peak Time of Average Pixel Value in Time Series Variation in Average Pixel Value

Time when the average pixel value in a graph obtained in the same manner as in (4) above shows a peak value ($t_{peak}$ in Figure 3)

In addition to those described above, another evaluation value may be obtained by calculating the average pixel value, maximum pixel value, minimum pixel value, and under curve area described in (1), (2), (3), or (4) for the entire liver region and obtaining a ratio of the value of each liver segment to the value of the whole liver region as the evaluation value. Further, an evaluation value obtained by obtaining time intensity variations in pixel values in the abdominal aorta and each liver segment using the analysis method described in Non-patent Document 3, and analyzing the time intensity variations by a deconvolution method may also be used. Further, it is possible to convert an evaluation value used in a known perfusion analysis for a CT image.

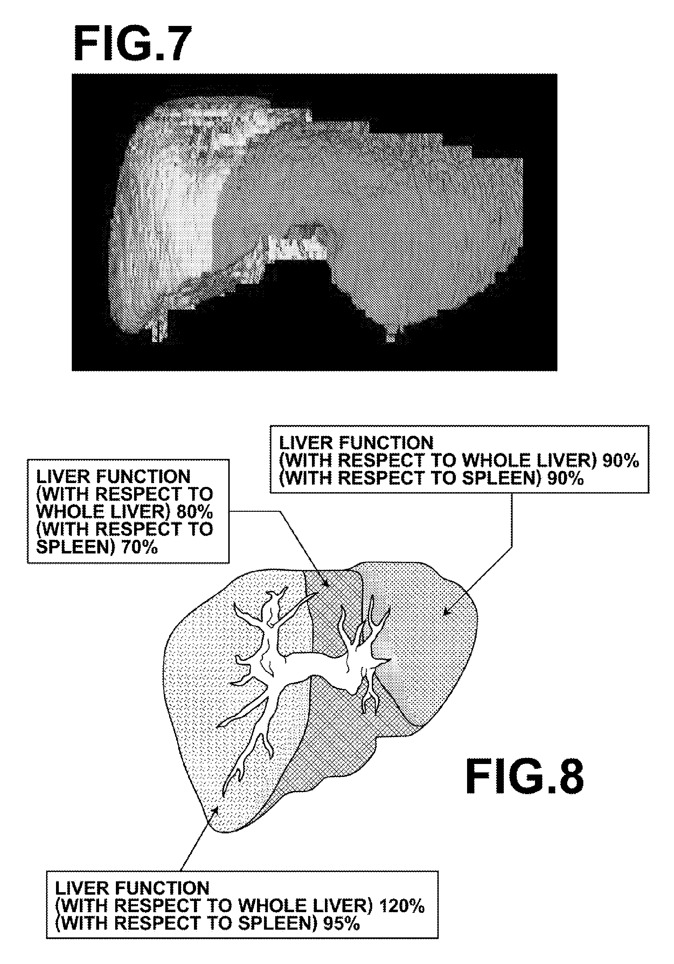

Display image generation unit 34 generates an image Img of the liver region based on evaluation values FS1, FS2, ----- of functional levels of respective liver segments. More specifically, the image Img is generated by performing a known volume rendering process on three-dimensional image data in which all pixels in each liver segment of the liver region have an evaluation value of the segment as the pixel value (Figures 7 and 8). Here, the image generation is performed in a manner that allows the difference in functional level with respect to each liver segment to be visually identifiable by allocating a different color to each value range of the evaluation value.

Display unit 35 is a display for displaying the image Img.

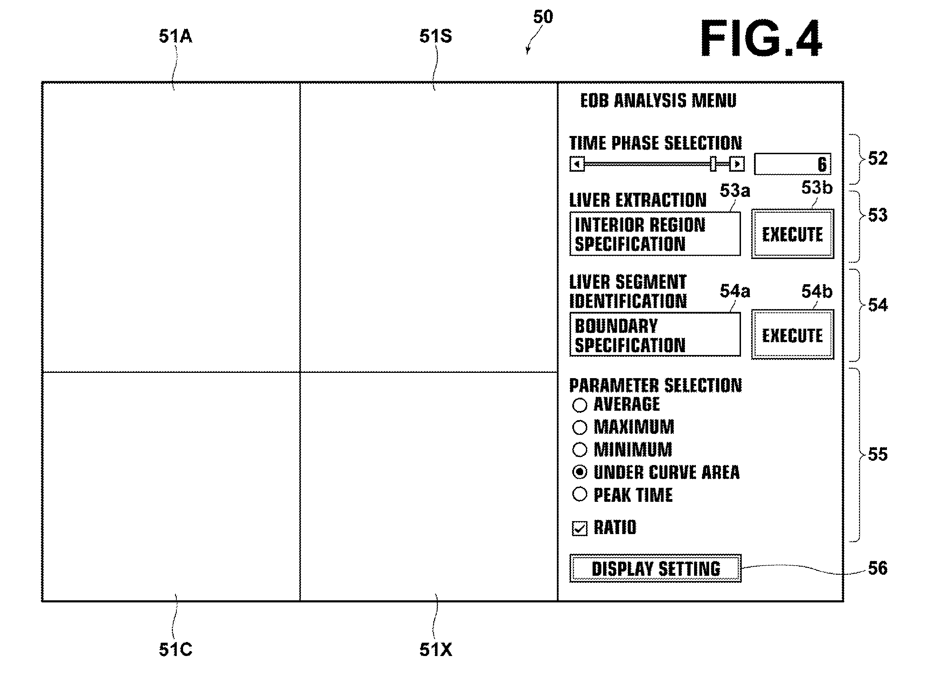

Next, a process flow of medical image diagnosis according to the first embodiment of the present invention will be described with reference to the block diagram shown in Figure 2 and screen examples shown in Figures 4 to 8.

First, when an examination order for liver function test using an EOB contrast agent is received at a terminal of an ordering system in an MRI room, an abdominal region of a test body of the examination order is imaged prior to being administered with the EOB contrast agent and a three-dimensional medical image $V_{t0}$ is generated. Then, the EOB contrast agent is administered to the test body, and identical imaging is performed at each time, for example, 20 sec, 1 min, 2 min, 5 min, 10 min, and 20 min after the administration of the EOB contrast agent, whereby three-dimensional medical images $V_{t1}$, -----, $V_{t6}$ are generated. The generated three-dimensional medical images $V_{t0}$, $V_{t1}$, -----, $V_{t6}$, i.e., three-dimensional dynamic images $V_{tn}$ are transferred to and stored in image storage server 2 and, at the same time, the examination order (radiology reading order) is transferred to a radiology reading room or an examination room in which image processing workstation 3 is installed.

When the examination order is selected in image processing workstation 3, the workstation obtains radiology reading target three-dimensional dynamic images $V_{tn}$ from image storage server 2 and analyzes the contents of the examination order to activate an application according to the examination order, i.e., an EOB analysis application in this case. Figure 4 schematically illustrates the configuration of initial screen 50 of the EOB analysis application. As shown in Figure 4, initial screen 50 includes 4 image display areas of axial image display area 51A, coronal image display area 51C, sagittal image displaye area 51S, and another image display area 51X. Initial screen 50 further includes, as a menu section for user operation, phase selection menu 52 for selecting a time phase of an image displayed in each of display areas 51A, 51C, 51S, liver extraction menu 53 serving as an interface for extracting a liver region, liver segment identification menu 54 serving as an interface for identifying each liver segment, parameter selection menu 55 for selecting a type of evaluation value (parameter) of functional level of each liver segment, and display setting button 56 for displaying a user interface for performing advanced settings of a displayed image.

It is assumed, here, that sectional images of three sections orthogonally intersecting at a predetermined position are reconstructed based on a hepatocyte phase image of reading target three-dimensional dynamic images $V_{tn}$ and displayed in axial image display area 51A, coronal image display area 51C, and sagittal image displaye area 51S respectively by the initial setting of the application. Preferably, the predetermined position is set to a position most appropriate for the user to specify the user set point when extracting a liver region. But an arrangement may be adopted in which the position of a section is moved according to a user operation, and sectional images are reconstructed at the moved position and the display is update. This allows the user to specify the user set point in a sectional image desired by the user.

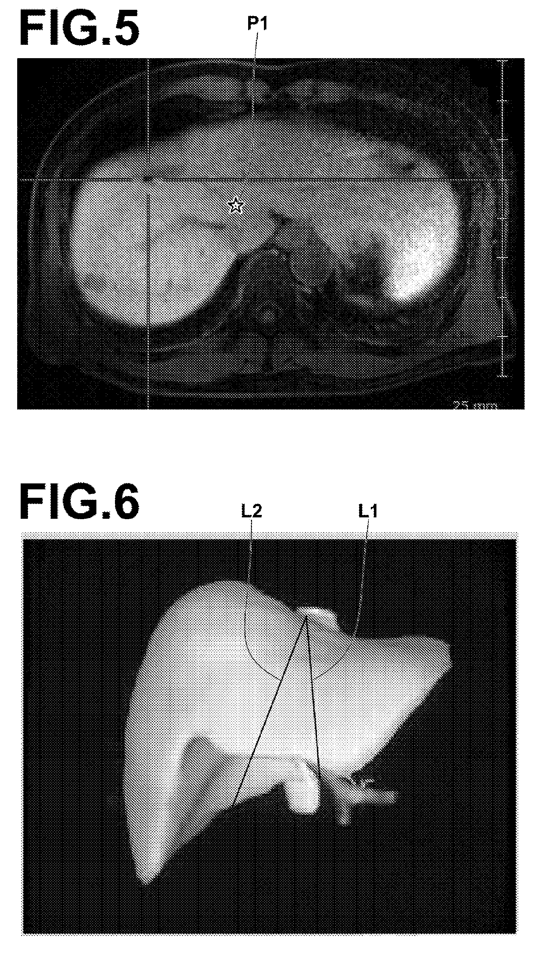

Next, an operation for extracting a liver region is performed by the user. More specifically, when interior region button 53a of liver extraction menu 53 is depressed by the user using the pointing device or the like, liver region extraction unit 31 changes the shape of the cursor of the pointing device to a specific shape and prompts the user to specify a user set point. When the pointing device is clicked on a point P1 in a liver region of an axial sectional image by the user, as shown in Figure 5, the point P is specified as the user set point. When execution button 53b of liver extraction menu 53 is further depressed by the user, the liver extraction processing described above is performed by the liver extraction unit 31 and a liver region $RL_{t6}$ is extracted from the three-dimensional image $V_{t6}$, whereby a volume rendering image of a liver region in the hepatocyte phase, as illustrated in Figure 6, is displayed in display area 51X. At this time, liver regions $RL_{t0}$ to $RL_{t5}$ in other time phases are also extracted in the background.

Next, an operation for identifying liver segments is performed by the user. More specifically, when boundary specifying button 54a of liver segment identification menu 54 is depressed by the user using the pointing device or the like, liver segment identification unit 32 changes the shape of the cursor of the pointing device to a specific shape and prompts the user to specify a boundary surface (line) of liver segments. For example, when the pointing device is clicked within the liver region of the volume rendering image shown in Figure 6, line sections L1 and L2 are specified as boundary surfaces of liver segments. When execution button 54b of liver segment identification menu 54 is further depressed by the user, the liver segment identification processing described above is performed by the liver segment identification unit 32, and three liver segments $RS1_{tn}$, $RS2_{tn}$, and $RS3_{tn}$ are identified in liver regions $RL_{tn}$ in each time phase in the example.

When a desired parameter is further selected by the user from the parameter selection menu 55, segment functional level calculation unit 33 calculates evaluation values (here, FS1, FS2, and FS3) of functional levels of respective segments by the calculation method described above based on the selected parameter. Here, as the under curve area in a time series variation in average pixel value described above is selected and a ratio checkbox is checked, a ratio of the under curve area of each liver segment to the under curve area of the entire liver is calculated.

Then, display image generation unit 34 generates a display image Img by performing the volume rendering process described above using calculated evaluation values FS1, FS2, and FS3. The generated image Img is displayed in display area 51X. Figures 7 and 8 illustrate display examples of image Img. Figure 7 shows an example volume rendering image in which two liver segments are identified. In the mean time, Figure 8 shows an example volume rendering image in which three liver segments are identified. In this case, the opacity setting is changed from that shown in Figure 7 to make the liver parenchyma portion semi-transparent by reducing the opacity and a color is allocated to blood vessels in the liver, whereby both the different color display for each liver segment and the display of blood vessel portion are achieved. Such display setting change may be made through a display image advanced setting interface displayed when display setting button 56 is depressed by the user.

As described above, according to the embodiment described above, from liver function angiographic images $V_{tn}$ obtained using an EOB contrast agent that produces contrast effects according to functional levels of a liver of the diagnostic target, evaluation values FS1, FS2, ----- representing functional levels of a plurality of liver segments $RS1_{tn}$, $RS2_{tn}$, ----- of liver regions $RL_{tn}$ are obtained by segment functional level calculation unit 33, an image Img based on the evaluation values is generated by display image generation unit 34, and the generated image Img is displayed in display unit 35. This allows easy and appropriate liver image diagnosis through the use of functional levels and segments of a liver. For example, when a target liver area for resection surgery is determined based on the image diagnosis of the liver, the functional level of the liver may be confirmed on a segment-by-segment basis, so that a determination may be made easily and appropriately as to which segment is to be a resection target.

A modification of the aforementioned embodiment will now be described.

In the embodiment described above, the position of the liver region may possibly be displaced from time phase to time phase due to body movement or respiration of the test body. Consequently, direct application of a liver region extracted or coordinates of a liver segment identified in one time phase to other time phases causes inaccurate extraction of a liver region or inaccurate identification of a liver segment in other time phases. Thus, it is preferable to perform positional alignment on three-dimensional dynamic images $V_{tn}$ to align the position of the liver region between the three-dimensional images of different time phases through translation, rotation, deformation, enlargement, reduction, or the like using any known rigid or non-rigid registration method (e.g., D. Rueckert et al., "Nonrigid Registration Using Free-Form Deformations: Application to Breast MR Images", IEEE Transactions on Medical Imaging, Vol. 18, No. 8, pp. 712-721, 1999) or an imaged region recognition process for axial projection images (e.g., U.S. Patent Application Publication No. 20080260226), thereby obtaining moving direction and amount for each pixel between time phases in advance. In this case, the liver region extracted or coordinates of a liver segment identified in one time phase may be converted using the moving direction and amount of each pixel with respect to another time phase, whereby the liver region or liver segment in another time phase may be identified accurately.

In the aforementioned embodiment, a plurality of liver segments is identified, and a functional evaluation value of each segment is visualized. But, an arrangement may be adopted in which segment identification and display of functional evaluation value are performed on a segment-by-segment basis, like identifying only one segment first, calculating a functional evaluation value for the segment, and displaying the segment in a color according to the evaluation value, then identifying another segment, calculating a functional evaluation value for the segment, and displaying the segment in a color according to the evaluation value, and so on.

In the embodiment described above, a description has been made of a case in which a ratio of each liver segment to the whole liver is used when calculating an evaluation value of functional level. But a ratio with respect to the other organ, for example, a spleen region in which the absorption of the EOB contrast agent occurs (Figure 8) may be used. For an MRI image, absolute signal values are meaningless and a relationship relative to the other region is important. Therefore, calculation of functional evaluation values using the other organ as the reference region, as described above, allows more appropriate functional evaluation. In this case, the spleen region may be extracted by a method identical to the extraction method of liver region described above.

In the embodiment described above, the display image Img is a pseudo three-dimensional image using volume rendering which allows functional evaluation of each liver segment from various angles by changing the position of the viewpoint or visual line. But the display image Img may be a two-dimensional projection image depending on the performance of the image processing workstation. Further, instead of displaying functional evaluation values in different colors or in addition to the color code display, actual evaluation values may be displayed in character, as shown in Figure 8, or an icon or the like may be used instead of character.

The EOB contrast agent used in the embodiment described above is an example contrast agent that produces contrast effects according to functional levels of a liver, and other contrast agents which produce identical effects, i.e., allowing acquisition of a liver function angiographic image in which functional levels of the liver are reflected as pixel levels may be used. Such other contrast agents may include, for example, SPIO contrast agent.

A second embodiment of the present invention is a case in which medical image diagnosis is performed using a liver function angiographic image that appropriately represents the function of a liver but does not clearly represents an anatomical structure of the liver and a morphological image that represents the anatomical structure of the liver. In the second embodiment, modality 1 in the hardware configuration of Figure 1 includes a SPECT unit and a CT unit. The SPECT unit is capable of performing dynamic imaging in which three-dimensional medical images $V2_{t1}$, $V2_{t2}$, ----- (hereinafter collectively referred to as $V2_{tn}$) of an abdominal/chest region (including heart and liver) are obtained at predetermined times after administration of asialo scinti. The three-dimensional dynamic images $V2_{tn}$ correspond to liver function angiographic images but, unlike the first embodiment, do not clearly represent an anatomical structure of a liver. Consequently, in the present embodiment, a three-dimensional morphological image V3 representing an anatomical structure of a liver is further obtained using the CT unit.

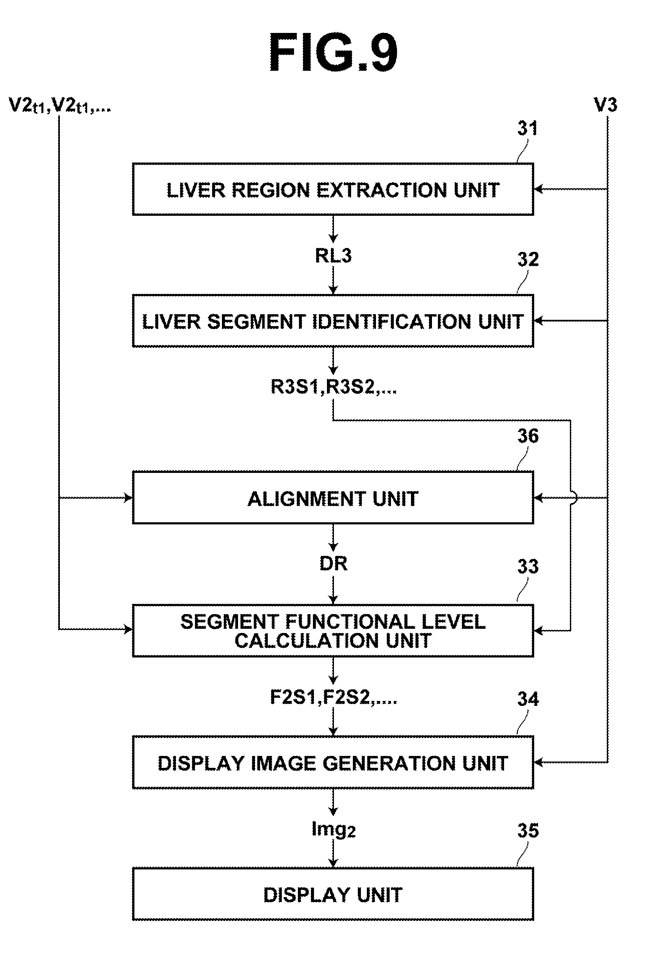

Figure 9 is a block diagram, illustrating a portion of the function of image processing workstation 3 relevant to the process of medical image diagnosis according to the second embodiment of the present invention. As shown in the drawing, the process of medical image diagnosis according to the second embodiment of the present invention is realized by liver region extraction unit 31, liver segment identification unit 32, alignment unit 36, segment functional level calculation unit 33, display image generation unit 34, and display unit 35. Each unit will now be described in detail.

Liver region extraction unit 31 and liver segment identification unit 32 obtain a liver region RL3 and a plurality of liver segments R3S1, R3S2, ----- from the three-dimensional morphological image V3 respectively in the same manner as in the first embodiment.

Alignment unit 36 aligns the positions of liver regions between the three-dimensional dynamic images $V2_{tn}$ and three-dimensional morphological image V3 using the non-rigid registration method described above and outputs a moving direction/amount DR of each pixel between the images.

Segment functional level calculation unit 33 identifies positions in the three-dimensional dynamic image $V2_{tn}$ (hereinafter, liver segments R2S1, R2S2, ----- of the three-dimensional dynamic image $V2_{tn}$) corresponding to liver segments R3S1, R3S2, ----- by converting coordinate values of liver segments R3S1, R3S2, of the three-dimensional morphological image V3 identified by liver segment identification unit 32 using the moving direction/amount DR of each pixel obtained by alignment unit 36, and calculates evaluation values F2S1, F2S2, ----- representing liver functional levels for the identified liver segments R2S1, R2S2, ----- of the three-dimensional dynamic image $V2_{tn}$ respectively.

Display image generation unit 34 generates an image $Img_2$ by performing a known volume rendering process on three-dimensional image data in which all pixels in each of liver segments R3S1, R3S2, ----- of the three-dimensional morphological image V3 have each of evaluation values F2S1, F2S2, ----- of functional levels of each of corresponding liver segments R2S1, R2S2, ----- of the three-dimensional dynamic images $V2_{tn}$ as the pixel value.

The generated image $Img_2$ is displayed on a display of display unit 35.

As described above, in the second embodiment of the present invention, liver segments R3S1, R3S2, ----- in a three-dimensional morphological image V3 representing an anatomical structure of a liver are identified and then liver segments R2S1, R2S2 ----- in three-dimensional dynamic images $V2_{tn}$, in which liver function is imaged, corresponding to liver segments R3S1, R3S2 ----- identified in the three-dimensional morphological image V3 are identified using a moving direction/amount DR of each pixel. Thus, even when three-dimensional dynamic images $V2_{tn}$ do not clearly represent the anatomical structure of a liver, this allows evaluation values F2S1, F2S2, ----- respectively representing functional levels of liver segments R2S1, R2S2, ----- to be obtained from the three-dimensional dynamic images $V2_{tn}$ by supplementing information of the anatomical structure of the liver by way of the three-dimensional morphological image V3.

In the embodiment described above, the combination of segment identification unit 32, alignment unit 36, and processing by segment functional level calculation unit 33 for identifying liver segments R2S1, R2S2, ----- in three-dimensional dynamic images $V2_{tn}$ corresponds to a segment identification means of the present invention.

In the embodiment described above, when imaging conditions, such as imaging body position, setting of coordinate axis for an image to be generated, and the like, are matched between the SPECT unit and CT unit, the alignment process by alignment unit 36 is not required and coordinate values of liver segments R3S1, R3S2,obtained from the three-dimensional morphological image V3 may be used directly for the three-dimensional dynamic images $V2_{tn}$.

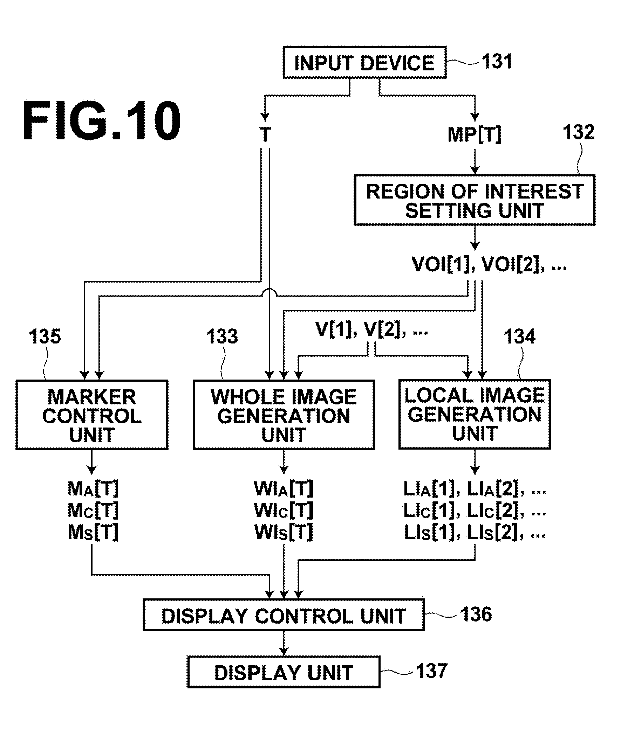

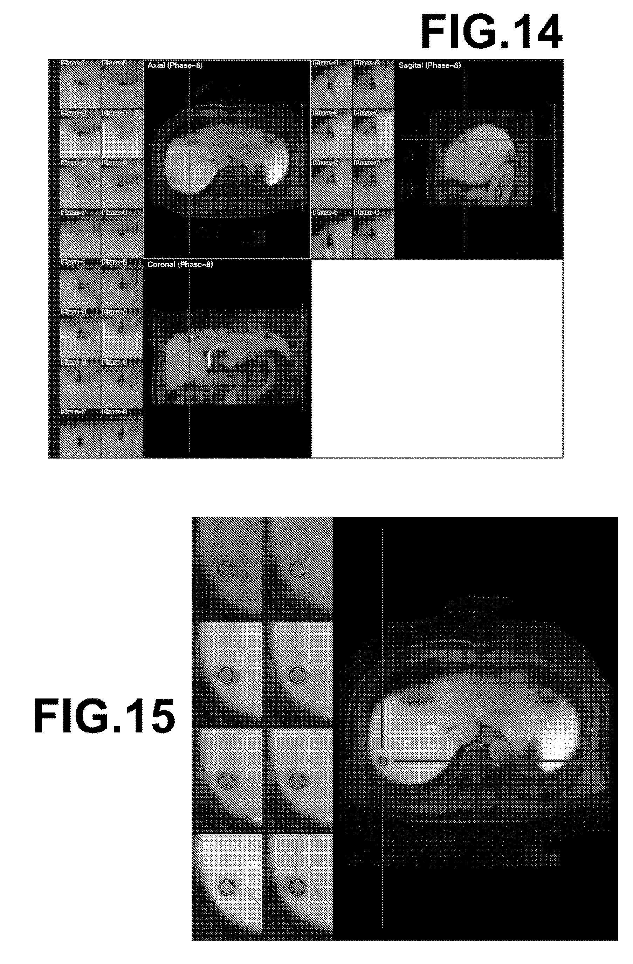

A medical image diagnostic system according to a third embodiment generates, based on liver angiographic images (V[1], V[2], -----, V[8] in Figure 10, which are, hereinafter, collectively referred to as V[t], in which t represents time phases) obtained in a plurality of time phases in the contrast examination using the EOB contrast agent described above, whole sectional images ($WI_A[T]\cdot WI_C[T]\cdot WI_S[T]$ in Figure 10, which are, hereinafter, collectively referred to as WI[T]) representing the whole liver in a time phase specified by the user, which is based on axial, coronal, and sagittal sections passing through a region of interest specified by the user, generates local sectional images ($LI_A[1]\cdot LI_C[1]\cdot LI_S[1]$, $LI_A[2]\cdot LI_C[2]\cdot LI_S[2]$, ----- in Figure 10, which are, hereinafter, collectively referred to as LI[t]), which are based on axial, coronal, and sagittal sections passing through the region of interest and corresponding regions of interest in other time phases for respective phases, and displays the whole sectional images WI[T] and local sectional images LI[t] side-by-side (Figure 14). Here, as an example of the plurality of time phases, it is assumed that three-dimensional liver angiographic images (voxel data) V[2], V[3] -----, V[8] are to be obtained 20 sec, 1 min, 2 min, 5 min, 10 min, 20 min, and 21 min after the administration of the contrast agent. Note that the elapsed time after administration of the EOB contrast agent is related to liver angiographic image in each phase obtained by the imaging as accessory information. Also note that the hardware configuration of the medical image diagnostic system of the present embodiment is identical to that of the first embodiment shown in Figure 1.

Figure 10 is a block diagram, illustrating a portion of the function of image processing workstation 3 relevant to the process of medical image diagnosis according to the third embodiment of the present invention. As shown in the drawing, the process of medical image diagnosis according to the third embodiment of the present invention is realized by input device 131, region of interest setting unit 132, whole image generation unit 133, local image generation unit 134, marker control unit 135, display control unit 136, and display unit 137. Each unit will now be described in detail. In the following description, the three-dimensional liver angiographic image is described as liver angiographic image.

Input device 131 is provided for the user to select a time phase T for which the whole sectional images $WI_A[T], WI_C[T], WI_S[T]$ are generated and displayed, and to specify the center positions MP[T] of regions of interest in the displayed whole sectional images $WI_A[T]$, $WI_C[T]$, $WI_S[T]$. In the present embodiment, the description will be made on the assumption that input device 131 is a pointing device such as a mouse. But the Input device 131 may be another device, such as a keyboard.

Region of interest setting unit 132 identifies the positions of regions of interest VOI[1], VOI[2], ----- VOI[8] in the liver angiographic images V[1], V[2], ----- in the respective time phases. More specifically, region of interest setting unit 132 sets a cubic area of certain pixels (voxels) corresponding to a predetermined length on each side centered on the position MP[T] in liver angiographic image V[T] in time phase T as a region of interest VOI[T] based on the center positions MP[T] of the regions of interest of the whole sectional images $WI_A[T], WI_C[T], WI_S[T]$. The length of one side of the cubic area is predetermined by taking into account a maximum possible displacement of the liver due to respiration and body movement of the test body under the contrast examination. Then, with respect to each of liver angiographic images in time phases other than the time phase T, an area having the same coordinate values as the region of interest VOI[T] is set as a region of interest.

Based on the time phase T specified by the user, whole image generation unit 133 reconstructs whole sectional images $WI_A[T] \cdot WI_C[T] \cdot WI_S[T]$ of axial, coronal, and sagittal sections passing through the center of region of interest VOI[T] in time phase T using a known MPR (Multi-planar Reconstruction) technique with the liver angiographic image V[T] as input.

With the liver angiographic images V[1], V[2], -----, V[8] in the respective time phase as input, local image generation unit 134 reconstructs local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$, which are based on axial, coronal, and sagittal sections passing through the center positions of the regions of interest VOI[1], VOI[2], -----, VOI[8] by performing a known MPR technique on image data representing inside of regions of interest VOI[1], VOI[2], -----, VOI[8] in the respective time phases.

Marker control unit 135 obtains, based on a time phase T specified by the user, position information of the region of interest VOI[T] in the time phase T and generates markers $M_A[T] \cdot M_C[T] \cdot M_S[T]$ for indicating the regions of interest in the whole sectional images $WI_A[T] \cdot WI_C[T] \cdot WI_S[T]$ base on the axial, coronal, and sagittal sections. A specific form of the marker will be described later.

Display control unit 136 controls display unit (display) to display the whole sectional images $WI_A[T] \cdot WI_C[T] \cdot WI_S[T]$, markers $M_A[T] \cdot M_C[T] \cdot M_S[T]$, and local sectional images $LI_A[1] \cdot LI_C[1] \cdot LI_d[1]$, $LI_A[2] \cdot LI_C[2] \cdot LI_S[2]$, -----, $LI_A[8] \cdot LI_C[8] \cdot LI_S[8]$ in a predetermined layout.

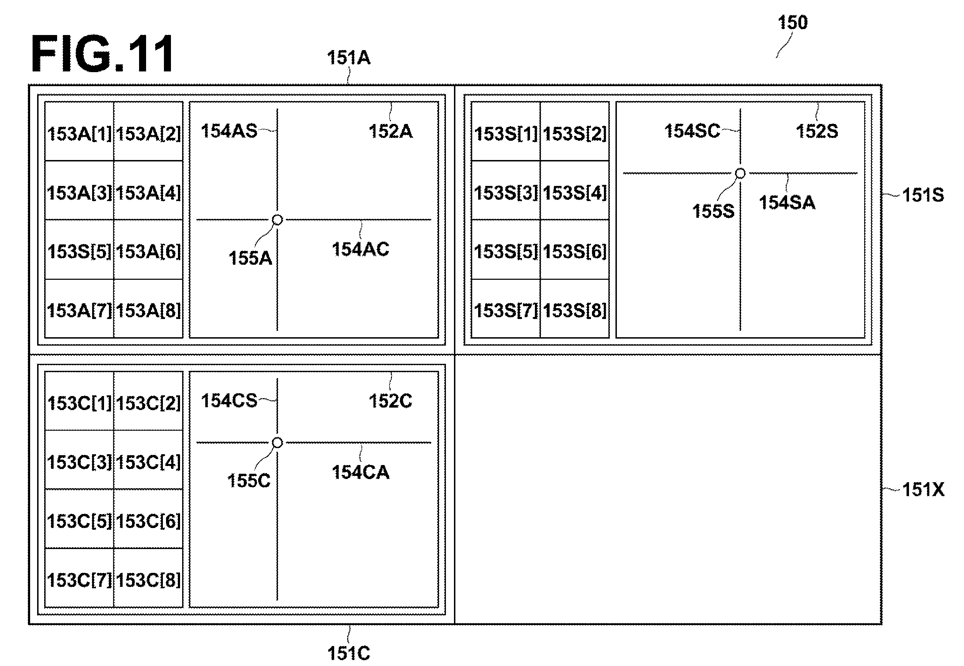

Figure 11 shows an example layout of screen 150 displayed on display unit 137. As shown in the drawing, display screen 150 is largely divided into axial image display area 151A, coronal image display area 151C, sagittal image display area 151S, and arbitrary image display area 151X. Axial image display area 151A includes whole image display area 152A where a whole sectional image $WI_A[T]$ based on an axial section is displayed and local image display areas 153A[1], 153A[2], -----, 153A[8] where local sectional images $LI_A[1]$, $LI_A[2]$, -----, $LI_A[8]$ based on the axial section in the respective time phases are displayed. Further, the marker $M_A[T]$ is displayed superimposed on the whole sectional image $WI_A[T]$. More specifically, the marker $M_A[T]$ is displayed as the intersection point (intersection line) between a straight line 154AS representing the sagittal section and a straight line 154 AC representing the coronal section passing through the center of the region of interest VOI[T] in the axial sectional image, i.e., as a point 155A representing the center of the region of interest VOI[T]. As described above, in the present embodiment, the center point of a region of interest corresponds to the intersection point of three sections of axial, coronal, and sagittal. As in axial image display area 151A, coronal image display area 151C/sagittal image display area 151S includes whole image display area 152C/152S where a whole sectional image $WI_C[T]/WI_S[T]$ based on a coronal/sagittal section is displayed and local image display areas 153C[1], 153C[2], -----, 153C[8]/153S[1], 153S[2], -----, 153S[8] where local sectional images $LI_C[1]$, $LI_C[2]$, -----, $LI_C[8]/LI_S[1]$, $LI_S[2]$, -----, $LI_S[8]$ based on the coronal/sagittal section in the respective time phases are displayed. The marker $M_C[T]$ in the whole image display area 152C of the coronal image display area 151C is displayed as the intersection point between a straight line 154CS representing the sagittal section and a straight line 154 CA representing the axial section passing through the center of the region of interest VOI[T] in the coronal sectional image, i.e., as a point 155C representing the center of the region of interest VOI[T]. The marker $M_S[T]$ in the whole image display area 152S of the sagittal image display area 151S is displayed as the intersection point between a straight line 154SC representing the coronal section and a straight line 154SA representing the axial section passing through the center of the region of interest VOI[T] in the sagittal sectional image, i.e., as a point 155S representing the center of the region of interest VOI[T]. Arbitrary image display area 151X is an area where any image can be displayed, and it is also possible to further divide the area to display a plurality of images.

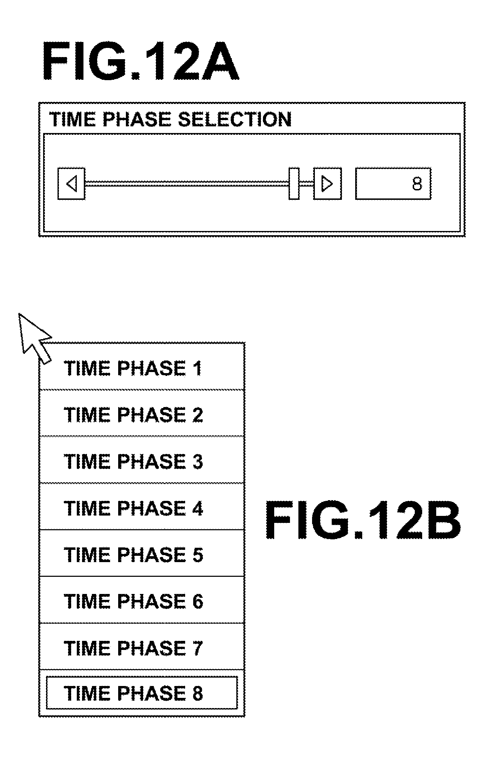

Figures 12A and 12B show specific examples of user interface for selecting the time phase T through input device 131. An interface that separately displays a phase selection window and accepts time phase selection through an operation of input device 131 to move the slide lever (e.g., a drag operation of the slide lever using a mouse), as shown in Figure 12A, or an interface that displays a phase selection menu through an operation of input device 131 (e.g., clicking the right mouse button) and accepts time phase selection through an operation of input device 131 (e.g., moving a mouse pointer to a desired time phase and clicking the left mouse button) may be conceivable. Another conceivable example is an interface that accepts time phase selection from local image display areas 153A[t], 153C[t], 153S[t] shown in Figure 11 through an operation of input device 131 for selecting a display area of desired time phase T (e.g., moving the mouse pointer to the display area of desired time phase and clicking the left mouse button).

In the mean time, a specific example of operation, which can be conceived, for changing the position of the center point MP[T] of a region of interest VOI[T] through input device 131 is a specifying operation using input device 131 at a desired position in the whole image display area 152A, 152C, or 152S shown in Figure 11. For example, a mouse double-click operation on a desired position, a drag operation for moving one of the straight lines 154AS, 154AC, 154CS, 154CA, 154SC, 154SA, which are markers in the whole image display areas 152A, 152C, 152S, or one of the intersection points 155A, 155C, 155S to a desired position.

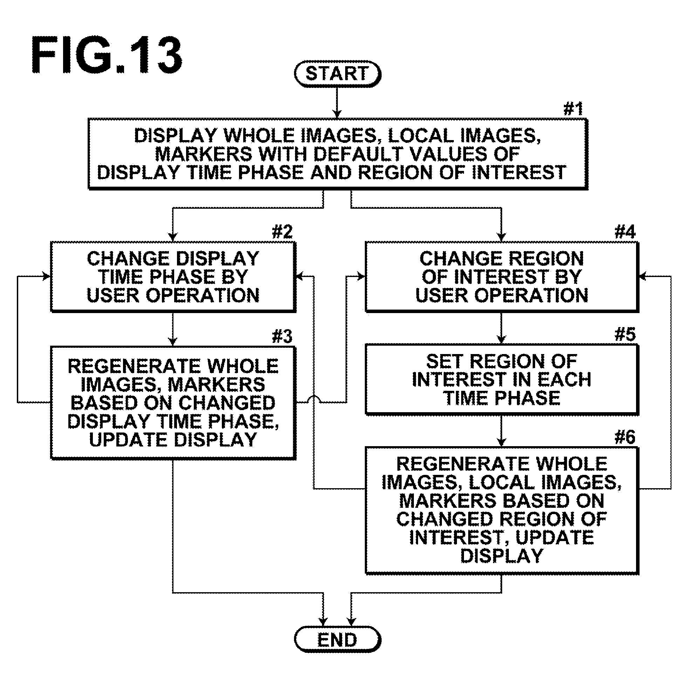

A process flow of liver image diagnosis using the medical image diagnostic system of the present embodiment will now be described. Figure 13 is a chart illustrating operation transitions in the medical image diagnosis of the present embodiment.

First, when an examination order for liver function test using an EOB contrast agent is received at a terminal of an ordering system in a MRI imaging room, an abdominal region of a test body of the examination order is imaged prior to being administered with the EOB and a liver angiographic image V[1] is generated. Then, the EOB contrast agent is administered to the test body, and identical imaging is performed at each time, for example, 20 sec, 1 min, 2 min, 5 min, 10 min, 20 min, and 21 min after the administration of the EOB contrast agent, whereby liver angiographic images V[2],V[8] are generated. The generated liver angiographic images V[2], ----- V[8] are transferred to and stored in image storage server 2 and, at the same time, the examination order (radiology reading order) is transferred to a radiology reading room or an examination room in which image processing workstation 3 is installed.

When the examination order is selected in image processing workstation 3, the workstation obtains a radiology reading target liver angiographic image V[T] from image storage server 2 and analyzes the contents of the examination order to activate an application program according to the examination order, i.e., an EOB analysis application in this case.

When the EOB analysis application is activated, a setting file for the application is read to obtain a default value $T_0$ of the time phase of a display target whole sectional image and a default value $MP[T_0]_0$ of the center point of a region of interest $VOI[T_0]$.

Region of interest setting unit 132 identifies the positions $VOI[t]_0$ of regions of interest in liver angiographic images V[t] in the respective time phases based on the default value $MP[T_0]_0$ of the center point. Then, based on the default value $T_0$ of the display target time phase and a default value $VOI[T_0]_0$ in the time phase To, whole image generation unit 133 reconstructs whole sectional images $WI[T_0]_0$ to be displayed first with the liver angiographic image $V[T_0]$ in the time phase $T_0$ as input, and marker control unit 135 generates markers $M[T_0]_0$ representing the position $VOI[T_0]_0$ of the region of interest. Based on regions of interest $VOI[t]_0$ in the respective time phases, local image generation unit 134 reconstructs local sectional images $LI[t]_0$ to be displayed first with liver angiographic images V[t] in the respective phases as input. Display control unit 136 causes display unit 137 to display the generated whole sectional images $WI[T_0]_0$, markers $M[T_0]_0$, and local sectional images $LI[t]_0$ in the layout shown in Figure 11 (#1).

The user observes the whole sectional images $WI[T_0]_0$ and local sectional images $LI[t]_0$ displayed on display unit 137. When a candidate lesion area suspicious of a lesion is not found in the images, the use may perform, as required, an operation for changing the display target time phase of whole sectional images (#2) or region of interest, i.e., position of the section of the whole sectional images and local sectional images (#4).

When an operation for changing the display target time phase from $T_0$ to $T_1$ is performed by the user through the interface shown in Figure 12A or 12B (#2), whole image generation unit 133 reconstructs, based on the changed time phase $T_1$ and region of interest $VOI[T_1]_0$ in the time phase $T_1$, whole sectional images $WI[T_1]_0$ with the liver angiographic image $V[T_1]$ in the time phase $T_1$ as input, marker control unit 135 generates markers $M[T_1]_0$ representing the position $VOI[T_1]_0$ of the region of interest, and display control unit 136 updates the display to whole sectional images $WI[T_1]_0$ and markers $M[T_1]_0$ (#3). Here, the display of the local sectional images $LI[t]_0$ is not updated since the position of the region of interest is not changed.

In the mean time, when the position of the center point of the region of interest $VOI[T_0]_0$ is changed from MP $[T_0]_0$ to $MP[T_0]_1$ by the user by moving a marker in the whole image display area 152A, 152C, or 152S shown in Figure 11 in the display screen based on the default values (#4), region of interest setting unit 132 identifies, based on the changed position $MP[T_0]_1$ of the center point, the positions $VOI[t]_1$ of regions of interest of liver angiographic images V [t] of the respective time phases (#5). Then, based on the display target time phase $T_0$ and the changed region of interest $VOI[T_0]_1$ in the time phase To, whole image generation unit 133 reconstructs whole sectional images $WI[T_1]$ with the liver angiographic image $V[T_0]$ as input and marker control unit 135 generates markers $M[T_0]_1$ representing the changed position $VOI[T_0]_1$ of the region of interest. Based on the changed regions of interest $VOI[t]_1$ in the respective time phases, local image generation unit 134 reconstructs local sectional images $LI[t]_1$ with liver angiographic images V[t] in the respective phases as input. Display control unit 136 causes display unit 137 to display the generated whole sectional images $WI[T_0]_1$, markers $M[T_0]_1$, and local sectional images $LI[t]_1$ in the layout shown in Figure 11 (#6) . As described above, when the position of the region of interest is changed, the entire display is updated to the whole sectional images $WI[T_0]_1$, markers $M[T0]_1$, and local sectional images $LI[t]_1$.

Thereafter, as illustrated by the arrow from step #3 to step #2 or #4 and the arrow from step #6 to step #2 or #4 in Figure 13, the user may repeat, as required, the operation for changing the target display time phase of the whole sectional images (#2) or for changing the position of region of interest (#4), and the display of the images and markers is appropriately updated according to the changing operation (#3, #5, #6) . If, for example, a candidate lesion area is found by the user, the user may, as required, change the display target time phase of the whole sectional images (#2) to update the display of the whole sectional images and markers (#3), move the marker such that the center of the candidate lesion area corresponds to the center (intersection of each section) of the region of interest (#4) to change the region of interest in each time phase (#5), and update the display of whole sectional images, markers, and local sectional images (#6) in order to display whole sectional images in a time phase appropriate for the observation of the candidate lesion area and adjacent areas thereof. Figure 14 shows an example screen in which whole sectional images WI[8] in time phase 8 and local sectional images LI[t] of a candidate lesion area in the images are displayed. As shown in Figure 14, functional levels of liver cells in the candidate lesion area and adjacent areas thereof can be observed from the whole sectional images WI[8] by the contrast effects on normal liver cells of the EOB contrast agent in a latter phase of the contrast examination (time phase 8, hepatocyte phase) and, at the same time, a time series variation of the candidate lesion area due to change from the angiographic effects of the EOB contrast agent in an initial phase (arterial phase) to contrast effects on normal liver cells in a latter phase may be observed from the local sectional images LI[t].

As described above, according to the third embodiment of the present invention, region of interest setting unit 132 respectively sets liver regions of interest VOI[1], VOI[2], -----, VOI[8], which are positionally correspond to each other between each time phase, in liver angiographic images V[1], V[2], ----- V[8] obtained in a contrast examination using an EOB contrast agent that reflects blood flow and function of a liver in an image. Then, based on the liver angiographic images V[1], V[2], ----- V[8] in the respective time phases, local image generation unit 134 generates, with respect to each time phase, local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$ representing regions of interest VOI[1], VOI[2], -----, VOI[8], and display control unit 136 causes the generated local sectional images with respect to each time phase $LI_A[1] \cdot LI_C[1] \cdot LI_S[1]$, $LI_A[2] \cdot LI_C[2] \cdot LI_S[2]$, -----, $LI_A[8] \bullet LI_C[8] \cdot LI_S[8]$ to be displayed side-by-side in one display screen. This allows easy performance of comparative reading of displayed local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$ with respect to each time phase and a time series variation in signal value of the regions of interest VOI[1], VOI[2], -----, VOI[8] in each time phase may be understood in detail and efficiently. That is, the embodiment of the present invention described above meets the demand of accurately understanding a time series variation in signal value of a region of interest in a plurality of time phases in a contrast examination using an EOB contrast agent and is extremely useful for image diagnosis of livers using the EOB contrast agent.

Further, whole image generation unit 133 generates whole sectional images $WI_A[T]$, $WI_C[T]$, $WI_S[T]$ representing an area that includes the liver region and the region of interest VOI[T] in the display target time phase T, and display control unit 136 causes the whole sectional images $WI_A[T]$, $WI_C[T]$, $WI_S[T]$ and the local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$ to be displayed at the same time. This allows simultaneous observation of a time series variation of the regions of interest VOI[1], VOI[2], -----, VOI[8] and appearance of adjacent areas thereof and hence more detailed diagnosis may be made.

In this case, marker control unit 135 causes markers $M_A[T] \cdot M_C[T] \cdot M_S[T]$ representing the region of interest VOI[T] to be displayed superimposed on the whole sectional images $WI_A[T]$, $WI_C[T]$, $WI_S[T]$. This allows the position of the region of interest VOI[T] in each of the whole sectional images $WI_A[T]$, $WI_C[T]$, $WI_S[T]$ to be identified easily, whereby image reading efficiency is improved.

Further, if an arrangement is adopted in which the markers $M_A[T] \cdot M_C[T] \cdot M_S[T]$ are made movable by the user using input device 131, the positions MP[T] of the markers $M_A[T] \cdot M_C[T] \cdot M_S[T]$ after the movement are detected, a region of interest VOI[T] is set in the liver angiographic image V[T], which is the source of the whole sectional images $WI_A[T]$, $WI_C[T]$, $WI_S[T]$, by region of interest setting unit 132, and regions of interest VOI[t] are set in liver angiographic images V[t] in time phases other than the time phase of the liver angiographic image V[T] in which the region of interest VOI[T] is set, the regions of interest VOI[1], VOI[2], -----, VOI[8] may be moved according to the moving operation of the markers. Then, whole sectional images $WI_A[T] \cdot WI_C[T] \cdot WI_S[T]$ representing the region of interest after moved in time phase T may be generated by whole image generation unit 133, local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$ representing the regions of interest VOI[1], VOI[2], ----- VOI[8] after moved in each phase may be generated by local image generation unit 134, and each generated image may be displayed through display control unit 136. That is, the regions of interest VOI[1], VOI[2]. ----- VOI[8] are moved in association with an operation, by the user, to move the markers $M_A[T] \cdot M_C[T] \cdot M_S[T]$, whereby the display may be switched to the whole sectional images $WI_A[T] \cdot WI_C[T] \cdot WI_S[T]$ and local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$ representing the regions of interest VOI[1], VOI[2], ----- VOI[8] after moved. Consequently, while observing whole sectional images $WI_A[T] \cdot WI_C[T] \cdot WI_S[T]$, the user may set a region of interest VOI[T] in the images and display local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$ at the point, whereby a time series variation in signal value of the portion where the region of interest VOI[T] is set may be observed at the same time and more detailed and flexible observation may be performed efficiently.

Still further, the liver angiographic images V[1], V[2], -----, V[8] are three-dimensional images and the regions of interest are three-dimensional regions VOI[1], VOI[2], ----- VOI[8], whole sectional images $WI_A[T] \cdot WI_C[T] \cdot WI_S[T]$ viewed from a plurality of directions are generated by whole image generation unit 133, so that the region of interest and a surrounding area thereof can be three-dimensionally observed easily, whereby more detailed diagnosis may be made. In addition, local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$ also viewed from a plurality of directions are generated by local image generation unit 134, so that the regions of interest VOI[1], VOI[2], ----- VOI[8] can be observed from a plurality of directions, whereby more detailed diagnosis may be made. For example, if an area appearing as a round lesion in local sectional images $LI_A[1]$, $LI_A[2]$, -----, $LI_A[8]$ based on the axial section also appears round in local sectional images $LI_C[1] \cdot LI_S[1]$, $LI_C[2] \cdot LI_S[2]$, -----, $LI_C[8] \cdot LI_S[8]$ based on sagittal and coronal sections, it can be determined that the area is highly likely a lesion, while if the area appears tubular in local sectional images of sagittal and coronal sections, the area is a blood vessel and may conclude that a cross-section of a blood vessel appeared like a lesion in the local sectional images of the axial section.

Further, in the embodiment described above, images representing an area greater than a maximum possible displacement between regions of interest VOI[1], VOI[2], -----, VOI[8] are used as the local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$. This may eliminate the problem that the region of interest is displaced outside of the local sectional image in a certain time phase due to displacement between time phases arising from the respiration or body movement of the test body, whereby observation accuracy of a time series variation of the region of interest may be improved.

A modification of the third embodiment will now be described. In the embodiment described above, region of interest setting unit 132 sets a region having the same coordinate values as those of a region of interest VOI[T] specified by the user in display target time phase T in each of the other time phases as a region of interest. With respect to liver angiographic images V[t] in the respective time phases, positional alignment may be performed between liver angiographic images in different time phases using the rigid or non-rigid registration method or the imaged region recognition process described above to obtain moving direction and amount for each pixel between time phases in advance. Then, the coordinate values of the region of interest specified in the display target time phase T may be converted using the moving direction and amount of each pixel with respect to another time phase, whereby coordinate values of a corresponding region of interest in another time phase may be obtained. As for the positional alignment, an arrangement may be adopted in which a drag/drop operation of, for example, each local image shown in Figure 14 allows the local image to be moved in a direction parallel to the screen, thereby allowing the user to manually perform a positional alignment and a manual correction for an automatic positional alignment result by the method described above. With respect to liver angiographic images V[t] in a plurality of different time phases, if positionally corresponding regions of interest VOI[t] are set based on image content characteristics in the manner as described above, regions of interest VOI[t] may be set at anatomically the same position in liver angiographic images V[t] in the respective time phases without being influenced by the displacement of the regions of interest VOI[t] between time phases arising from the body movement, respiration, or the like of the test body. This may eliminate the need, as in the embodiment described above, to predetermine the size of a region of interest (in a local sectional image) by taking into account a maximum possible displacement of the region of interest and may resolve the problem that a region of interest is not included in a local sectional image in a certain time phase, whereby observation accuracy of a time series variation of the region of interest may be improved.

Further, in the embodiment described above, marker control unit 135 displays, in a superimposing manner, a marker representing a region of interest only in a whole sectional image, but the maker may be superimposed on a region of interest of a local sectional image in each time phase. Figure 15 illustrates a display example in which a marker is indicated in each of local images. Figure 15 shows only axial image display area 151A, but the marker may be displayed, in a superimposing manner, on each local image in coronal image display area 151C, sagittal image display area 151S, and arbitrary image display area 151X. The marker can take a various forms and, for example, it may be an arrow, a text comment, or the like.

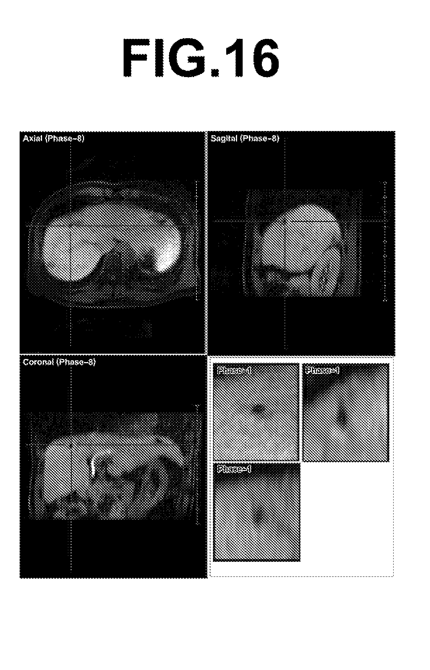

Still further, in the embodiment described above, local sectional images in all time phases are displayed side-by-side in one screen to facilitate comparative reading. If a large display screen can not be obtained due to physical constraints of the display and the like, if a local sectional image representing a region of interest of wide area needs to be observed, or if a region of interest needs to be enlarged and observed, however, an arrangement may be adopted in which axial · coronal · sagittal whole sectional images in one time phase are displayed in axial image display area 151A, coronal image display area 151C, and sagittal image display area 151S respectively, and only axial·coronal·sagittal local images in one phase are displayed side-by-side in arbitrary image display area 151X, as shown by way of example in Figure 16, and local sectional images in the respective time phases are sequentially switched and displayed in a time series manner through an operation of input device 131 (e.g., clicking on one of the local sectional images and performing a mouse wheel operation) . Such display mode allows a time series variation of the region of interest to be captured like a motion picture.

Further, in the in the embodiment described above, liver angiographic images V[t] in all time phases obtained by modality 1 (MRI system) through dynamic imaging are used as targets for the generation and display of whole sectional images and local sectional images, but a time phase of interest selection unit for selecting a generation/display target time phase may further be provided in the configuration of Figure 10. The time phase of interest selection unit may be a unit that performs automatic selection based on a predetermined selection condition or manual selection through a user operation. A specific example of automatic selection is a method in which an acquisition timing for a generation/display target time phase (e.g., a time elapsed from the administration of a contrast agent) is provided in advance, as a selection condition, in a setting file or the like and a liver angiographic image in a time phase corresponding to the selection condition is selected by referring to accessory information of acquisition timing related to each of liver angiographic images V[t]. A specific example of manual selection is a method in which a representative image (e.g., axial sectional image at a predetermined position) reconstructed from each of liver angiographic images V[t] obtained by modality 1 and acquisition timing information of the image are list-displayed, and a generation/display target time phase is selected by the user using input device 131. The selectable generation/display target time phase achieved in the manner as described above allows only the images in a time phase required for diagnosis to be observed, thereby contributing to diagnostic efficiency improvement.

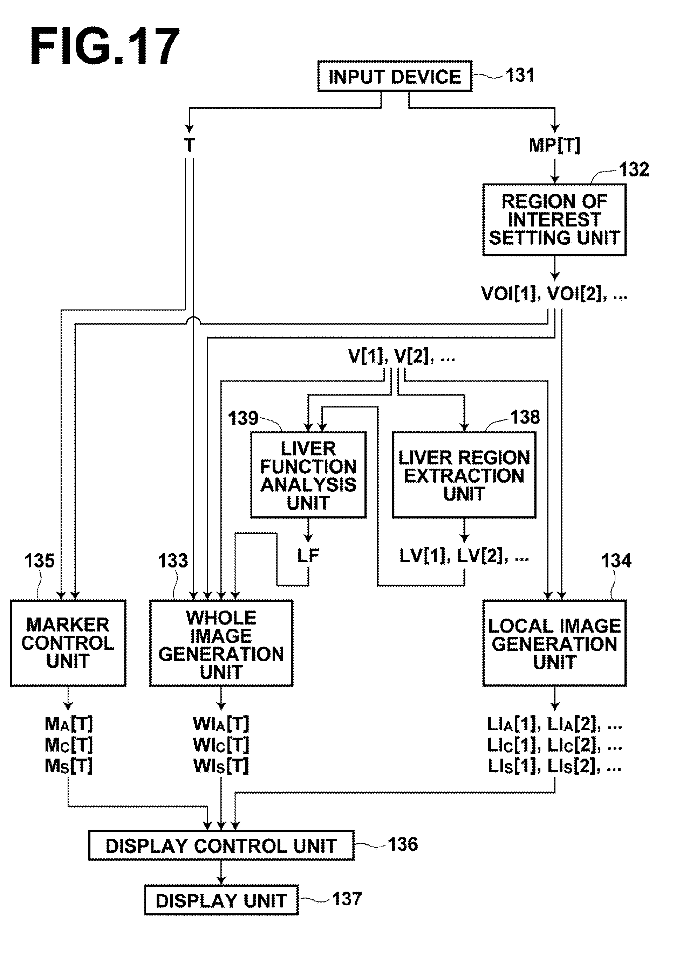

Still further, in the in the embodiment described above, a whole sectional image WI[T] is generated from a liver angiographic image V[T] in one display target time phase T selected by the user and displayed. But, a functional image in which the time axis is compressed is generated as a whole sectional image using liver angiographic images in a plurality of time phases obtained in a contrast examination. Figure 17 is a block diagram illustrating such a modification. In addition to the configuration of Figure 10, the configuration of Figure 17 includes liver region extraction unit 138 and liver function analysis unit 139.

Liver region extraction unit 138 extracts liver regions LV[1], LV[2], ----- LV[8] respectively from liver angiographic images in a plurality of different time phases V[1], V[2], ----- V[8] using a method identical to that of liver region extraction unit 31 in the first embodiment. With respect to liver angiographic images V[1], -----, V[7], each of the liver regions LV [1], -----, LV[7] may be extracted from each of the liver angiographic images using a method identical to that described above or an area of each of the liver angiographic images with coordinate values corresponding to those of the liver region LV[8] may be regarded as each of the liver regions LV[1], -----, LV[7]. Further, as described above, positions of the liver regions LV[1], -----, LV[7] may be identified by performing coordinate transformation on the liver region LV[8] based on positional alignment results between different time phases using the aforementioned rigid or non-rigid registration method, the imaged region recognition process, or the like.

Based on liver angiographic images in a plurality of different time phases V[1], V[2], ----- V[8] and liver regions LV[1], -----, LV[8] in the respective time phases, liver function analysis unit 139 divides the liver region into small areas with a unit of predetermined pixels (voxels), and calculates a liver function evaluation value LF representing a functional level of the liver with respect to each small area. The small area may be, for example, a cubic area with a length of about 3 to 5 pixels (voxels) on a side. But, the liver function evaluation value LF may be calculated with respect to each pixel (voxel), i.e., for each cubic area with a length of one pixel (voxel) on a side.

Here, specific examples of the liver function evaluation value LF may include average pixel value, maximum average pixel value, minimum average pixel value, under curve area in time series variation in average pixel value, peak time of average pixel value in time series variation in average pixel value described in the first embodiment calculated with respect to each small area instead of each liver segment and ratios of these values to the values of entire liver region.

Further, as in the first embodiment, an evaluation value obtained by obtaining time intensity variations in pixel values in the abdominal aorta and each small area using the analysis method described in Non-patent Document 3, and analyzing the time intensity variations by a deconvolution method may also be used. Further, it is possible to convert an evaluation value used in a known perfusion analysis for a CT image. Further, when calculating liver function evaluation values, a ratio with respect to the other organ, for example, a spleen region in which the absorption of the EOB contrast agent occurs (Figure 8) may be used. For an MRI image, absolute signal values are meaningless and a relationship relative to the other region is important. Therefore, calculation of functional evaluation values using the other organ as the reference region, as described above, allows more appropriate functional evaluation. In this case, the spleen region may be extracted by a method identical to the extraction method of liver region described above.

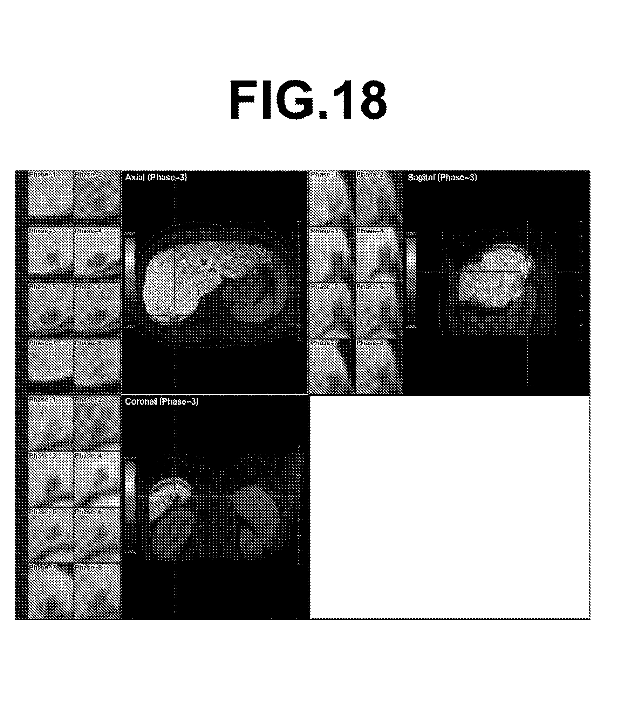

In the present modification, whole image generation unit 133 generates whole sectional images $WI_A[T] \cdot WI_C[T] \cdot WI_S[T]$ based on liver angiographic images in a plurality of different time phases V[1], V[2], -----, V[8] and calculated liver function evaluation values LF. More specifically, it is possible to generate whole sectional images (sectional morphological images) $WI_A[T] \cdot WI_C[T] \cdot WI_S[T]$ of axial, coronal, and sagittal sections passing through the center of region of interest VOI[T] in a predetermined display target time phase T, then to generate sectional images (functional sectional image) based on the axial, coronal, and sagittal sections passing through the center described above by regarding a liver functional level LF of each small area as a three-dimensional functional image, and to generate an image by superimposing the generated two types of images. Figure 18 illustrates an example image generated in the manner as described above and displayed on display unit 137 by display control unit 136. As shown in Figure 18, a functional sectional image is displayed superimposed on a morphological sectional image in a liver area for which the liver function evaluation value LF is calculated. The liver function evaluation values which are pixel values in a functional sectional image are mapping imaged in a manner that allows a difference in liver functional level to be visually recognizable by allocating a different color to each liver function evaluation value range. Note that the local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$ are identical to those in the embodiment described above.

As described above, according to the present modification, whole image generation unit 133 generates whole sectional images $WI_A[T] \cdot WI_C[T] \cdot WI_S[T]$ further using function evaluation values LF of a liver region obtained by liver region extraction unit 138 and liver function analysis unit 139. This allows a functional image representing a function of the liver and a local morphological image representing a region of interest to be displayed at the same time, thereby contributing to diagnostic efficiency improvement.

In the modification described above, a liver is divided into comparatively small areas and a liver function evaluation value LF is calculated for each area, but the liver may be divided into comparatively large areas like Couinaud's eight segments. Diagnosis or surgery of a liver is generally performed on a segment-by-segment basis classified according to control areas of hepatic artery, portal vein, and hepatic vein. In the case in which a target region for resection surgery is determined, the determination should be made on a segment-by-segment basis. Thus, the simultaneous display of whole sectional images $WI_A[T] \cdot WI_C[T] \cdot WI_S[T]$ representing a functional level of each segment and local sectional images $LI_A[1] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$ representing a region of interest which is a lesion candidate in the liver appropriately meets such needs of medical sites.

As for a specific method of dividing a liver into segments, a manual or automatic division method identical to that of segment identification unit 32 in the first embodiment may be used.

Further, in the embodiment described above, an image obtained under a different imaging condition, e.g., a T1 weighted image or a T2 weighted image prior to the administration of a contrast agent may be displayed in arbitrary image display area 151X of Figure 11 on grounds that the contrast observation of such image is useful for distinguishing a liver tumor. More specifically, it is known that a dysplastic nodule becomes hyperintense in a T1 weighted image and becomes hypointense in a T2 weighted image, a well-differentiated liver tumor becomes isointense to slightly hyperintense in a T1 weighted image and becomes isointense to slightly hypointense in a T2 weighted image, and a moderately-differentiated liver tumor becomes hypointense in a T1 weighted image and becomes hyperintense in a T2 weighted image. Simultaneous observation of T1 weighted image and T2 weighted image prior to administration of the EOB contrast agent shown in arbitrary image display area 151X, and liver angiographic images after administration of the EOB contrast agent shown in other display areas 151A, 151C, and 151S allows more accurate liver image diagnosis.

Alternatively, an image generated by other image processing may be displayed in arbitrary image display area 151X of Figure 11. A specific image example to be displayed may be an image img (Figures 7 and 8) representing a function evaluation value with respect to each liver segment generated in the first embodiment. In this way, simultaneous display of whole sectional images $WI_A[T] \cdot WI_C[T] \cdot WI_S[T]$, like those shown in Figure 14, representing the morphology of the entire liver region, local sectional images $LI_A[L] \cdot LI_C[1] \cdot LI_S[1]$, $LI_A[2] \cdot LI_C[2] \cdot LI_S[2]$, -----, $LI_A[8] \cdot LI_C[8] \cdot LI_S[8]$ representing morphology of a region of interest which is a lesion candidate in the liver, and an image like that shown in Figure 7 or 8 representing a function evaluation value with respect to each liver segment allows a simultaneous observation of a time series variation of the region of interest and around the region of interest in addition to a liver function evaluation with respect to each liver segment which is important in liver image diagnosis as described above. This may further meet the needs of medical sites and is extremely effective for improving liver image diagnostic efficiency.

Further, the EOB contrast agent used in the third embodiment described above is an example contrast agent that causes blood flow and function of a liver to be reflected in an image. It may be another type of contrast agent having an identical effect, i.e., a contrast agent that allows acquisition of a liver angiographic image in which blood flow and function of the liver is reflected.

Still further, in the third embodiment described above, whole sectional images are displayed simultaneously with local sectional images. But only the local sectional images are displayed or the local sectional images and an image representing a function with respect to each liver segment shown in Figure 7 or 8 may be displayed simultaneously. Otherwise, an arrangement may be adopted in which these display modes are switched and displayed automatically or through user operation.

Further, in the third embodiment described above, whole sectional images and local sectional images are interlocked by the same section position, but the section position may be different.

Still further, the region of interest may be displayed separately by visualizing the time series variation in the liver evaluation value. A specific visualization form is a graph representing a time series variation in the average pixel value of a region of interest in each time phase, as shown in Figure 3.

It should be appreciated that any modification to the system configuration, process flow, module structure, and the like in each aforementioned embodiment without departing from the spirit of the present invention is included in the technical scope of the present invention.

For example, in the aforementioned embodiments, the description has been made that each process shown in Figures 2, 9, and 10 is performed in one image processing workstation 3. But a configuration may by adopted in which the processes are allocated to a plurality of image processing workstations and performed cooperatively.

Further, each embodiment described above is for illustrative purposes only and should not be construed as limiting the technical scope of the present invention. [Supplementary Explanation: Details of Liver Region Extraction Units 31 and 138]

Hereinafter, details of a liver region extraction method will be described by quoting the specification of Japanese Patent Application No. 2008-050615. In order to keep the explanation short and simple, an extraction method from a two-dimensional image will be described first and then an extraction method from a three-dimensional image will be described.

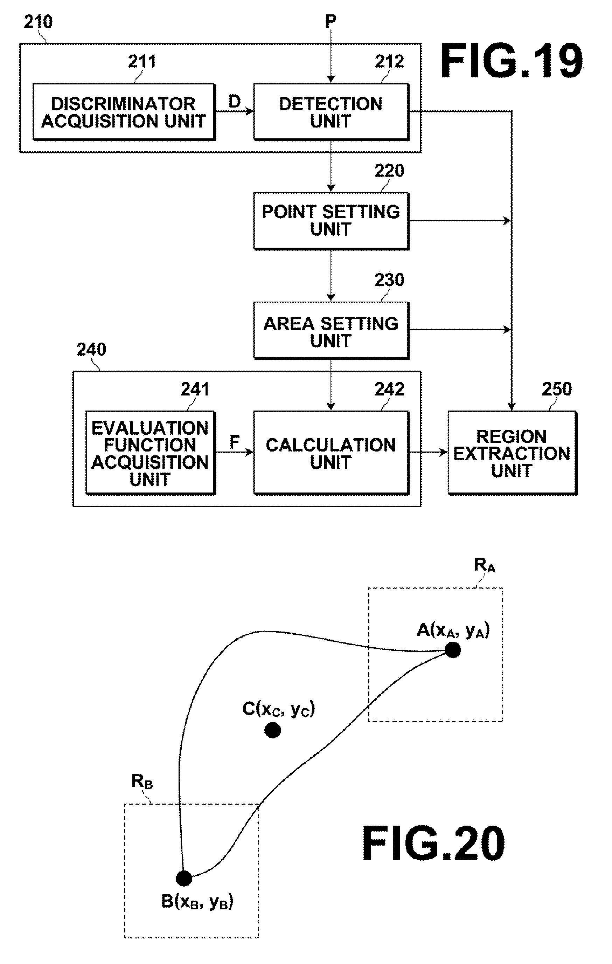

As illustrated in Figure 19, liver region extraction unit 31 or 138 is a unit for extracting a liver region from a medical image P and includes reference point detection unit 210, point setting unit 220, area setting unit 230, contour likelihood calculation unit 240, region extraction unit 250, and the like.

Reference point detection unit 210 is a unit that has machine-learned, for each of a plurality of sample images in which a reference point located on a contour of a liver region and identifiable based on a pixel value distribution of an adjacent area is known, a pixel value distribution of adjacent area of each of a pixel representing the reference point and a pixel representing a point other than the reference point, and detects a reference point in the medical image P based on the result of the machine learning. Reference point detection unit 210 includes discriminator acquisition unit 211 and detection unit 212.

Here, the reference point is a point which is set according to the type of a target region desired to be extracted from an input image, and there is no restriction on the number. In the present embodiment, for example, either one or both of point A and point B located at angulated portions of a liver contour of a generally smooth curve, as shown in Figure 20, are used as the reference point.

Discriminator acquisition unit 211 is a unit for acquiring a discriminator D, by providing a plurality of sample images, each including a liver region, and machine learning pixel value distributions of adjacent areas of each of a pixel representing a reference point and a pixel representing a point other than the reference point in each sample image in advance, that determines whether or not each pixel in each sample image is a pixel representing the reference point based on a pixel value distribution of an adjacent area of the pixel as described, for example, in U.S. Patent Application Publication No. 20060098256.

The discriminator D acquired in the manner as described above may be used for determining whether or not each pixel is a pixel representing the reference point in any medical image. Where a liver region is extracted using two or more reference points (e.g., reference points A and B), a discriminator D is acquired for each reference point.

Preferably, the adjacent area of each pixel is an area large enough to determine whether or not the pixel is the reference point based on the pixel value distribution in the adjacent area, such as the direction and magnitude of changing in pixel value of each pixel, and the like. Further, the adjacent area may be an area centered on a target pixel or an area that includes the target pixel away from the center.

Figure 20 illustrates an example of adjacent areas $R_A$ and $R_B$ of reference points A and B. Here, each adjacent area, by way of an example, has a rectangular shape, but the adjacent area may have various shapes including a circular shape, an oval shape, and the like. Further, a pixel distribution of only some of the pixels in the adjacent area may be used for the machine learning described above.

Further, Adaboosting Algorithm, Neural Network, SVM (Support Vector Machine), and the like may be used for acquiring the discriminator D.

Detection unit 212 is a unit for detecting reference points A and B in a medical image P by scanning the discriminator D acquired by discriminator acquisition unit 211 on the medical image P. If a discrimination area T, which seemingly includes the target area, is set in the medical image P by area setting unit 230, to be described later, before the reference point detection is performed by detection unit 212, the reference point detection may be performed by scanning the discriminator D only on the discrimination area T set by area setting unit 230 or a portion of the medical image P which includes the discrimination area T.

Point setting unit 220 is a unit for setting an arbitrary point C (seed point) in a liver area of the medical image P. It may be, for example, a unit that sets a position on the medical image P specified by operator input through the keyboard or pointing device of liver region extraction unit 31 or 138 as the arbitrary point or it may be a unit that sets, assuming that each point in a liver region automatically detected by a conventional target region detection method is given a certain mass, a position of the gravity center of the region as the arbitrary point.

Further, if reference points A, B are detected by reference point detection unit 210 before the arbitrary point C is set by point setting unit 220, the arbitrary point C ($x_C$, $y_C$) may be set by Formula (1) below using the reference point A ($x_A$, $y_A$) and reference point B ($x_B$, $y_B$) based on the anatomical positional relationship between the liver region and reference points A and B.

$$x_C = (x_A \times 1 + x_B \times 2) 3$$

$$y_C = (y_A \times 1 + y_B \times 2) 3 \qquad\qquad (1)$$

The arbitrary point C may be a point set at an approximate center of the liver region or a point set at a position away from the center of the liver region. Area setting unit 230 is a unit for setting a discrimination area T, which seemingly includes the entire liver region, in the medical image P. It may be, for example, a unit that sets an area of the medical image P specified by operator input through the keyboard or pointing device of liver region extraction unit 31 or 138 as the discrimination area T, or it may be a unit that automatically sets an area, which is larger than a possible size of the liver region, centered on the point C set by point setting unit 220 as the discrimination area T. This limits the area of the region of interest in the medical image P and the subsequent processing may be performed quickly.

The discrimination area T may have various shapes, as the circumferential shape, including a rectangular shape, a circular shape, an oval shape, and the like.

Contour likelihood calculation unit 240 is a unit for calculating a contour likelihood of each pixel in the discrimination area T set by area setting unit 230 based on pixel value information of adjacent pixels of the target pixel. Contour likelihood calculation unit 240 includes evaluation function acquisition unit 241 and calculation unit 242.

Evaluation function acquisition unit 241 is a unit for acquiring an evaluation function F, by providing a plurality of sample images, each including a liver region, and machine learning pixel value information of adjacent pixels of each of a pixel representing a point on a contour of the liver region and a pixel representing a point other than the contour in each sample image in advance as a characteristic amount of the target pixel, that evaluates whether or not each pixel in each sample image is a pixel representing the contour based on the characteristic amount of the pixel.

More specifically, through the use of pixel value information of adjacent pixels of each pixel, for example, a combination of pixel values of a plurality of different pixels in an area of $5 \times 5$ pixels in the horizontal and vertical directions centered on the target pixel, a plurality of weak classifiers for determining whether or not the target pixel is a pixel representing the contour is successively generated until the evaluation function F, which is a combination of all of the weak classifiers, has a desired performance for evaluating whether or not each pixel in each sample image is a pixel representing the contour as described, for example, in U.S. Patent Application Publication No. 20080044080.

The evaluation function F acquired in the manner as described above may be used for evaluating whether or not each pixel is a pixel representing a contour of a liver region in any medical image.

Also, when acquiring the evaluation function F, machine learning processes, such as Adaboosting Algorithm, Neural Network, SVM (Support Vector Machine), and the like may be used.

Calculation unit 242 is a unit for calculating, using the evaluation function F, a contour likelihood of each pixel, based on the characteristic amount of the target pixel in the discrimination area T.

Region extraction unit 250 is a unit for extracting a liver region from the discrimination area T using the arbitrary point C, reference point S, and contour likelihood of each pixel. When dividing the discrimination area T into a liver region and a background region by a graph cuts method described, for example, in Y.Y. Boykov and M-P. Jolly, "Interactive Graph Cuts for Optimal Boundary & Region Segmentation of Objects in N-D Images", Proceedings of "International Conference on Computer Vision", Vol. I, pp. 105-112, 2001 or U.S. Patent No. 6,973,212, region extraction unit 250 extracts the liver region from the discrimination area T such that the boundary between the liver region and background region is ensured to pass through the reference points A and B located on the contour of the liver region.

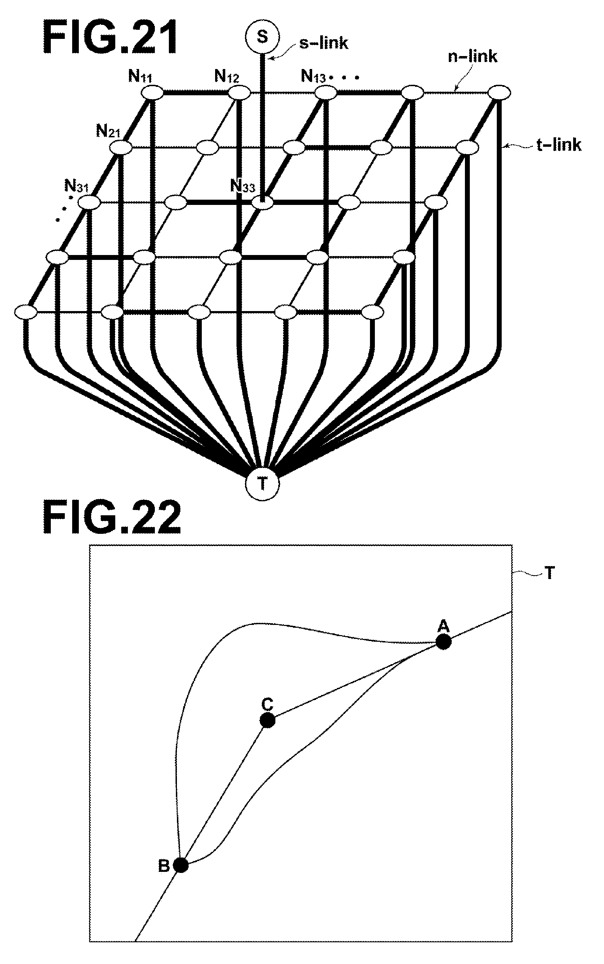

More specifically, a graph like that shown in Figure 21 is created first. As illustrated in Figure 21, the graph includes node $N_{ij}$ (i = 1, 2, -----, j = 1, 2, -----) representing each pixel in the discrimination area T, nodes S and T representing labels that each pixel can possibly take (liver region and background region in the present embodiment), n-links, each linking adjacent nodes of pixels, an s-link linking the node $N_{ij}$ representing each pixel and the node S representing the liver region, and a t-link linking the node $N_{ij}$ representing each pixel and the node T representing the background region.

Here, with respect to the n-links, the node $N_{ij}$ representing each pixel has four links extending toward four adjacent nodes, and there are two links between adjacent nodes, each extending to the other node. Here, the four links extending from a node $N_{ij}$ to four adjacent nodes represent a probability that the pixel represented by the node is in the same area as the four adjacent pixels and the probability is obtained by a contour likelihood of the pixel. More specifically, if the contour likelihood of the pixel represented by the node $N_{ij}$ is not greater than a preset threshold value, each of the links is given a maximum value of probability, while if the contour likelihood is greater than the threshold value, each of the links is given a probability such that the greater the contour likelihood the smaller the probability. For example, when a maximum value of probability is 1000, if the contour likelihood of the pixel represented by the node $N_{ij}$ is not greater than the threshold value (zero), each of the four links extending from the node to four adjacent nodes is given a value of 1000, while if the contour likelihood is greater than the threshold value (zero), each link is given a value calculated by (1000 - (contour likelihood/maximum value of contour likelihood) $\times$ 1000). Here, the maximum value of contour likelihood refers to a maximum value of all values of contour likelihood calculated by calculation unit 242 for pixels in the discrimination area.

The s-link linking the node $N_{ij}$ representing each pixel and node S representing a liver region is a link representing a probability that each pixel is a pixel included in the liver region. The t-link linking the node $N_{ij}$ representing each pixel and node T representing a background region is a link representing a probability that each pixel is a pixel included in the background region. If information of which of the regions, liver region or background region, each pixel represents is already provided, these probabilities are given according to the provided information.

More specifically, the arbitrary point C is a point set in a liver region, so that a large probability value is given to the s-link linking the node $N_{33}$ representing the point C and node S representing the liver region, as illustrated in Figure 21. The discrimination area T set with reference to an arbitrary point set in a liver region so as to include the liver region normally includes the liver region and a background region around the liver region. Therefore, it is assumed that each pixel on the periphery of the discrimination area T is a pixel representing a background, and a large probability value is given to each t-link linking each of nodes $N_{11}$, $N_{12}$, --, $N_{15}$, -----, $N_{21}$, -----, $N_{25}$, -----, $N_{31}$, -----, each representing each peripheral pixels.

Further, as illustrated in Figure 22, each pixel located in the portions between the reference point A and point C and between the reference point B and point C of the entire line segments extending to directions passing through the reference points A and B from the point C set by point setting unit 220 can be determined to be a pixel presents inside of the liver region. Therefore, a large probability value is given to the s-link linking the node representing each such pixel and the node S representing the liver region. On the other hand, each pixel located in the portion extending from the reference point A in a direction opposite to the point C and the portion extending from the reference point B in a direction opposite to the point C can be determined to be a pixel presents outside of the liver region. Therefore, a large probability value is given to the t-link linking the node representing each such pixel and the node T representing the background region.

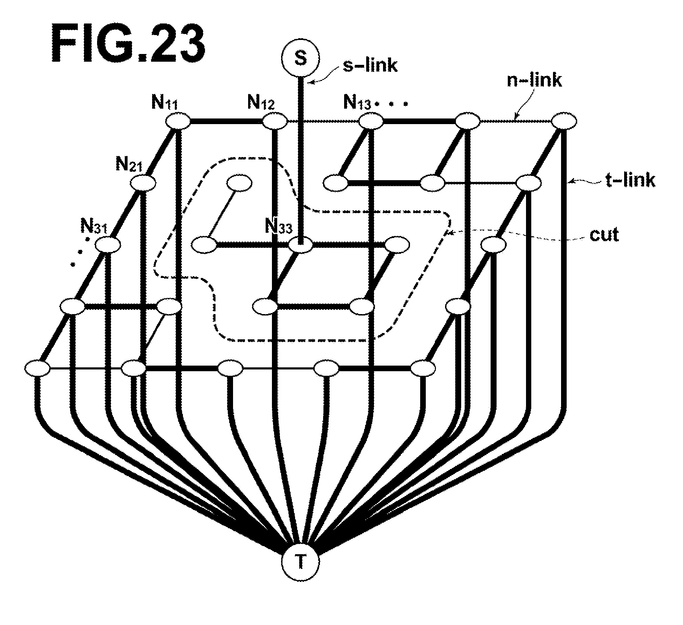

Then, as the liver region and background region are exclusive regions to each other, an appropriate link of all of the n-links, s-links, and t-links is cut as shown, for example, by a dotted line in Figure 23 to separate the node S from the node T, thereby dividing the discrimination area into a liver region and a background region and extracting the liver region. Here, an optimum regional division may be made by performing such cutting as to the total probability value of all of the n-links, s-links, and t-links to be cut becomes a minimum.

Here, the description has been made of a case in which a liver region is extracted using a graph cuts method, but instead of the graph cuts method, other methods may be used to extract the liver region, such as the method as described, for example, in U.S. Patent Application Publication No. 20080044080 in which a contour of a liver region is determined using a dynamic programming approach.

Next, example processing performed by the aforementioned configuration when extracting a liver region from a medical image will be described.

First, detection unit 212 detects reference points A and B in a medical image P using discriminators $D_A$ and $D_B$, acquired in advance by discriminator acquisition unit 211, capable of discriminating whether or not each pixel in an arbitrary medical image is a pixel representing either one of reference points A and B. Then, point setting unit 220 sets an arbitrary point C ($x_C$, $y_C$) (seed point) in a target region of the medical image P by Formula (1) shown above using the reference point A ($x_A$, $y_A$) and reference point B ($x_B$, $y_B$). Next, area setting unit 230 sets a discrimination area T which seemingly includes the entire target region in the medical image P. Then, calculation unit 242 calculates a contour likelihood of each pixel in the discrimination area T using an evaluation function F, acquired in advance by evaluation function acquisition unit 241, capable of evaluating whether or not each pixel in an arbitrary medical image is a pixel representing a contour of a liver region. Finally, region extraction unit 250 extracts the target region from the discrimination area T by, for example, a graph cuts method based on the arbitrary point C set by point setting unit 220 and the contour likelihood of each pixel calculated by calculation unit 242 such that the contour of the liver region is ensured to pass through the reference points A and B, whereby the processing is completed.

According to the embodiment described above, an arbitrary point is set in a target region of an input image, a discrimination area which seemingly includes the entire target region is set in the input image, a contour likelihood of each pixel in the discrimination area is calculated based on pixel value information of adjacent pixels of the target pixel, and when extracting the target region from the input image based on the set arbitrary point and the contour likelihood calculated for each pixel, a machine learning is performed, using a plurality of sample images in which a reference point located on a contour of a target region of the same type as the target region in the input image and identifiable based on a pixel value distribution of an adjacent area is known, for learning a pixel value distribution of adjacent area of each of a pixel representing the reference point and a pixel representing a point other than the reference point, and a reference point in the input image is detected based on the result of the machine learning, and the target region is extracted further based on the detection result. This ensures a contour of the target region to be determined so as to pass through the reference point detected as a point on a correct contour of the target region, whereby target region extraction performance may be improved.

In the embodiment described above, the description has been made of a case in which liver region extraction unit 31 or 138 of the present invention is applied to extract a target region from a two-dimensional input image, but they can also be used to extract a target region from a three-dimensional input image.

For example, when determining a contour of a liver region in a three-dimensional medical image, a point at an angulated portion of a liver contour of a generally smooth curve is used as a reference point, as in the liver region detection from a two-dimensional image.

Discriminator acquisition unit 211 acquires a discriminator, by providing a plurality of three-dimensional sample images, each including a liver region, and machine learning pixel value distributions of adjacent areas of each of a voxel representing a reference point and a voxel representing a point other than the reference point in each sample image in advance, that determines whether or not each voxel in each sample image is a voxel representing the reference point based on a pixel value distribution of an adjacent area of the voxel. Preferably, the adjacent area of each voxel is a three-dimensional area large enough to determine whether or not the voxel is the reference point based on the pixel value distribution in the adjacent area, such as the direction and magnitude of changing in pixel value of each voxel, and the like. Detection unit 212 detects the reference point by scanning the discriminator acquired by discriminator acquisition unit 211 on the three-dimensional medical image.

Point setting unit 220 sets an arbitrary point C, as a point in a three-dimensional coordinate system, in the liver region of the three-dimensional medical image, and area setting unit 230 sets a three-dimensional discrimination area T which seemingly includes the entire liver region in the three-dimensional medical image. Here, the three-dimensional discrimination area T may have various shapes, as the circumferential shape, including a hexahedron shape, a spherical shape, and the like.

Further, evaluation function acquisition unit 241 acquires an evaluation function F, by providing a plurality of sample images, each including a liver region, and machine learning pixel value information of adjacent voxels of each of a voxel representing a point on a contour of the liver region and a voxel representing a point other than the contour in each sample image in advance, capable of evaluating whether or not each voxel in an arbitrary three-dimensional medical image is a voxel representing a contour of a liver region. Here, as the pixel value information of adjacent voxels, for example, a combination of pixel values of a plurality of different voxels in a cubic area of $5 \times 5 \times 5$ voxels in the X axis, Y axis, and Z axis directions respectively centered on the target voxel. Then, calculation unit 242 calculates, using the evaluation function F, a contour likelihood of each voxel in the discrimination area T, i.e., an evaluation value of whether or not each voxel in the discrimination area T is a voxel representing a contour based on a characteristic amount of each voxel in the discrimination area T.

When dividing the three-dimensional discrimination area T into a liver region and a background region by a three-dimensional graph cuts method described, for example, in U. S. Patent No. 6973212, region extraction unit 250 extracts the liver region from the discrimination area T such that the boundary between the liver region and background region is ensured to pass through the reference point detected by detection unit 212, whereby the processing is completed.

**Claims**

1. A medical image diagnostic apparatus, comprising:

   a segment functional level information obtaining means (33) for obtaining, from a liver function angiographic image which is obtained after a predetermined time from administration to a test body of a contrast agent that produces a contrast effect according to a functional level of a liver and which represents a functional level of the liver of the test body, segment functional level information representing a functional level of at least one segment of the liver of the test body; and
   a segment functional level presentation means (34) for presenting the obtained segment functional level information.

2. The medical image diagnostic apparatus of claim 1, **characterized in that**:

   the segment functional level information obtaining means is a means that obtains segment functional level information of each of a plurality of segments of the liver of the test body; and
   the segment functional level presentation means is a means that presents the segment functional level information with respect to each of the plurality of segments.

3. The medical image diagnostic apparatus of claim 1 or 2, **characterized in that**:

   the liver function angiographic image is a three-dimensional image or images representing a time series variation in a three-dimensional space; and
   each segment is a three-dimensional region.

4. The medical image diagnostic apparatus of any of claims 1 to 3, further comprising a segment identification means (32) for identifying each segment of the liver of the test body.

5. The medical image diagnostic apparatus of claim 4, **characterized in that** the segment identification means is a means that identifies each segment of the liver of the test body in a morphological image representing an anatomical structure of the liver of the test body, and identifies each segment in the liver function angiographic image corresponding to each segment identified from the morphological image as each segment of the liver.

6. The medical image diagnostic apparatus of any of claims 1 to 5, **characterized in that**:

    the liver function angiographic image further includes image information of a region of the test body other than the liver; and
    the apparatus further comprises a liver region extraction means (31) for extracting a region of the liver from the liver function angiographic image.

7. The medical image diagnostic apparatus of any of claims 1 to 6, **characterized in that**:

    the liver function angiographic image further includes image information of a reference region of the test body other than the liver; and
    the segment functional level information obtaining means is a means that obtains the functional level of the segment as a relative relationship with respect to a functional level of the reference region.

8. The medical image diagnostic apparatus of claim 7, further comprising a reference region extraction means for extracting the reference region.

9. The medical image diagnostic apparatus of claim 7 or 8, **characterized in that** the reference region is a spleen region of the test body.

10. The medical image diagnostic apparatus of any of claims 1 to 9, further comprising:

    a region of interest setting means (132) for setting, with respect to each of images of two or more time phases of interest among liver angiographic images obtained in a plurality of time phases in a contrast examination using the contrast agent, a region of interest in the liver of test body positionally corresponding to each other between each of the time phases of interest;
    a local image generation means(134)for generating a local image representing the region of interest with respect to each of the time phases of interest based on each of the liver angiographic images of the time phases of interest; and
    a display control means (136) for causing the generated local image with respect to each of the time phases of interest to be displayed in a manner that allows comparative reading.

11. The medical image diagnostic apparatus of any of claims 1 to 10, **characterized in that** the liver function angiographic image is an image in which a normality level of liver function of the test body is reflected as a magnitude of pixel value.

12. The medical image diagnostic apparatus of any of claims 1 to 11, **characterized in that** the liver function angiographic image is an image obtained by MRI.

13. The medical image diagnostic apparatus of claim 12, **characterized in that** the contrast agent is a contrast agent that includes gadoxetate sodium (Gd-EOB-DTPA) as an active ingredient.

14. A medical image diagnostic method, comprising the steps of:

    obtaining, from a liver function angiographic image which is obtained after a predetermined time from administration to a test body of a contrast agent that produces a contrast effect according to a functional level of a liver and which represents a functional level of the liver of the test body, segment functional level information representing a functional level of at least one segment of the liver of the test body; and
    presenting the obtained segment functional level information.

15. A computer readable recording medium on which is recorded a program for causing a computer to perform the steps of:

    obtaining, from a liver function angiographic image which is obtained after a predetermined time from administration to a test body of a contrast agent that produces a contrast effect according to a functional level of a liver and which represents a functional level of the liver of the test body, segment functional level information representing a functional level of at least one segment of the liver of the test body; and
    presenting the obtained segment functional level information.

# FIG.1

MODALITY

IMAGE STORAGE SERVER

IMAGE PROCESSING WS

# FIG.2

$V_{t0} \sim V_{t6}$

| LIVER REGION EXTRACTION UNIT | 31 |

$RL_{t0} \sim RL_{t6}$

| LIVER SEGMENT IDENTIFICATION UNIT | 32 |

$RS1_{t0} \sim RS1_{t6}$
$RS2_{t0} \sim RS2_{t6}$

| SEGMENT FUNCTIONAL LEVEL CALCULATION UNIT | 33 |

FS1,FS2,....

| DISPLAY IMAGE GENERATION UNIT | 34 |

Img

| DISPLAY UNIT | 35 |

# FIG.3

# FIG.4

**EOB ANALYSIS MENU**

**TIME PHASE SELECTION** — 52
◄ ──────── ► [ 6 ]

**LIVER EXTRACTION** 53a 53b — 53
[ INTERIOR REGION SPECIFICATION ] [ EXECUTE ]

**LIVER SEGMENT IDENTIFICATION** 54a 54b — 54
[ BOUNDARY SPECIFICATION ] [ EXECUTE ]

**PARAMETER SELECTION** — 55
○ AVERAGE
○ MAXIMUM
○ MINIMUM
◉ UNDER CURVE AREA
○ PEAK TIME
☑ RATIO

[ DISPLAY SETTING ] — 56

50

51A  51S  51C  51X

EP 2 290 611 A1

# FIG.5

P1

# FIG.6

L2    L1

# FIG.7

LIVER FUNCTION
(WITH RESPECT TO WHOLE LIVER) 90%
(WITH RESPECT TO SPLEEN) 90%

LIVER FUNCTION
(WITH RESPECT TO
WHOLE LIVER) 80%
(WITH RESPECT TO
SPLEEN) 70%

# FIG.8

LIVER FUNCTION
(WITH RESPECT TO WHOLE LIVER) 120%
(WITH RESPECT TO SPLEEN) 95%

# FIG.9

V2$_{t1}$,V2$_{t1}$,...                                      V3

```
                                                    31
┌────────────────────────────────────────────┐
│       LIVER REGION EXTRACTION UNIT           │◄──
└────────────────────────────────────────────┘

                      RL3                      32
┌────────────────────────────────────────────┐
│      LIVER SEGMENT IDENTIFICATION UNIT       │◄──
└────────────────────────────────────────────┘

                  R3S1,R3S2,...
                                               36
┌────────────────────────────────────────────┐
│              ALIGNMENT UNIT                  │◄──
└────────────────────────────────────────────┘

                      DR                       33
┌────────────────────────────────────────────┐
│        SEGMENT FUNCTIONAL LEVEL              │◄──
│          CALCULATION UNIT                    │
└────────────────────────────────────────────┘

                 F2S1,F2S2,....                34
┌────────────────────────────────────────────┐
│       DISPLAY IMAGE GENERATION UNIT          │◄──
└────────────────────────────────────────────┘

                     Img2                      35
┌────────────────────────────────────────────┐
│               DISPLAY UNIT                   │
└────────────────────────────────────────────┘
```

# FIG.10

INPUT DEVICE — 131

T

MP[T] — 132

REGION OF INTEREST SETTING UNIT

VOI[1], VOI[2], ...

135

133

V[1], V[2], ...

134

MARKER CONTROL UNIT

WHOLE IMAGE GENERATION UNIT

LOCAL IMAGE GENERATION UNIT

$M_A[T]$
$M_C[T]$
$M_S[T]$

$WI_A[T]$
$WI_C[T]$
$WI_S[T]$

$LI_A[1], LI_A[2], ...$
$LI_C[1], LI_C[2], ...$
$LI_S[1], LI_S[2], ...$

DISPLAY CONTROL UNIT — 136

DISPLAY UNIT — 137

# FIG.11

# FIG.12A

TIME PHASE SELECTION

◁ ▷ 8

# FIG.12B

TIME PHASE 1

TIME PHASE 2

TIME PHASE 3

TIME PHASE 4

TIME PHASE 5

TIME PHASE 6

TIME PHASE 7

TIME PHASE 8

# FIG.13

```
                    ( START )
                        │
                        ▼                              #1
┌──────────────────────────────────────────────────────┐
│         DISPLAY WHOLE IMAGES, LOCAL IMAGES,            │
│            MARKERS WITH DEFAULT VALUES OF              │
│      DISPLAY TIME PHASE AND REGION OF INTEREST         │
└──────────────────────────────────────────────────────┘
         │                                    │
         ▼                  #2                 ▼                  #4
┌──────────────────────┐          ┌──────────────────────┐
│   CHANGE DISPLAY      │          │    CHANGE REGION      │
│    TIME PHASE BY      │          │    OF INTEREST BY     │
│   USER OPERATION      │          │    USER OPERATION     │
└──────────────────────┘          └──────────────────────┘
         │                  #3                 │                  #5
         ▼                                     ▼
┌──────────────────────┐          ┌──────────────────────┐
│  REGENERATE WHOLE     │          │    SET REGION OF      │
│  IMAGES, MARKERS      │          │   INTEREST IN EACH    │
│  BASED ON CHANGED     │          │     TIME PHASE        │
│ DISPLAY TIME PHASE,   │          └──────────────────────┘
│   UPDATE DISPLAY      │                     │             #6
└──────────────────────┘                     ▼
                                   ┌──────────────────────┐
                                   │  REGENERATE WHOLE     │
                                   │ IMAGES, LOCAL IMAGES, │
                                   │  MARKERS BASED ON     │
                                   │ CHANGED REGION OF     │
                                   │ INTEREST, UPDATE      │
                                   │     DISPLAY           │
                                   └──────────────────────┘

                        ( END )
```

## FIG.14

## FIG.15

# FIG.16

# FIG.17

INPUT DEVICE —131

T

MP[T]

REGION OF INTEREST SETTING UNIT —132

VOI[1], VOI[2], ...

V[1], V[2], ...

139— LIVER FUNCTION ANALYSIS UNIT

138 LIVER REGION EXTRACTION UNIT

LF

LV[1], LV[2], ...

135 MARKER CONTROL UNIT

133 WHOLE IMAGE GENERATION UNIT

134 LOCAL IMAGE GENERATION UNIT

$M_A[T]$
$M_C[T]$
$M_S[T]$

$WI_A[T]$
$WI_C[T]$
$WI_S[T]$

$LI_A[1], LI_A[2], ...$
$LI_C[1], LI_C[2], ...$
$LI_S[1], LI_S[2], ...$

DISPLAY CONTROL UNIT —136

DISPLAY UNIT —137

# FIG.18

## FIG.19

210

211
DISCRIMINATOR
ACQUISITION UNIT

D

P

212
DETECTION
UNIT

220
POINT SETTING
UNIT

230
AREA SETTING
UNIT

240

241
EVALUATION
FUNCTION
ACQUISITION
UNIT

F

242
CALCULATION
UNIT

250
REGION
EXTRACTION
UNIT

$R_A$

$A(x_A, y_A)$

$C(x_C, y_C)$

$R_B$

$B(x_B, y_B)$

## FIG.20

# FIG.21

# FIG.22

# FIG.23

Europäisches
Patentamt

European
Patent Office

Office européen
des brevets

# EUROPEAN SEARCH REPORT

Application Number

EP 10 17 3819

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AKIHIRO TANIMOTO ET AL: "Superparamagnetic iron oxide-enhanced MR imaging for focal hepatic lesions: a comparison with CT during arterioportography plus CT during hepatic arteriography", JOURNAL OF GASTROENTEROLOGY, SPRINGER-VERLAG, TO, vol. 40, no. 4, 1 April 2005 (2005-04-01), pages 371-380, XP019373194, ISSN: 1435-5922, DOI: DOI:10.1007/S00535-005-1553-8 | 1,2, 11-15 | INV. G06T7/00 A61B5/055 A61K49/06 G01R33/563 A61B6/00 |
| Y | * the whole document * | 2-10 | |
| Y | WO 2007/053676 A2 (EDDA TECHNOLOGY INC [US]; ZENG XIAOLAN [US]; MA FENG [US]; WEI GUO-QIN) 10 May 2007 (2007-05-10) * the whole document * | 2-5 | |
| Y | BE VAN BEERS, M LACROSSE, J JAMART, C GRANDIN, JF GIGOT, Y HORSMANS, R DEMEURE AND J PRINGOT: "Detection and segmental location of malignant hepatic tumors: comparison of ferumoxides-enhanced gradient-echo and T2-weighted spin- echo MR imaging", AMERICAN JOURNAL OF ROENTGENOLOGY, vol. 168, March 1997 (1997-03), pages 713-717, XP002613612, * the whole document * | 6-9 | TECHNICAL FIELDS SEARCHED (IPC) G06T A61B A61K G01R |
| Y | US 2005/201601 A1 (SUN YING [US] ET AL) 15 September 2005 (2005-09-15) * the whole document * | 10 | |

-/--

The present search report has been drawn up for all claims

| Place of search | Date of completion of the search | Examiner |
|---|---|---|
| Munich | 9 December 2010 | Anscombe, Marcel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

# EUROPEAN SEARCH REPORT

**Application Number**

EP 10 17 3819

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KEVIN C CHAN ET AL: "Dynamic contrast-enhanced MRI of ocular biotransport in normal and hypertensive eyes", ENGINEERING IN MEDICINE AND BIOLOGY SOCIETY, 2008. EMBS 2008. 30TH ANNUAL INTERNATIONAL CONFERENCE OF THE IEEE, IEEE, PISCATAWAY, NJ, USA, 20 August 2008 (2008-08-20), pages 835-838, XP031508084, ISBN: 978-1-4244-1814-5 * the whole document * | 10 | |
| X | HWANG E H ET AL: "Preoperative assessment of residual hepatic functional reserve using 99mTc-DTPA-galactosyl-human serum albumin dynamic SPECT", JOURNAL OF NUCLEAR MEDICINE, SOCIETY OF NUCLEAR MEDICINE, RESTON, VA, US, vol. 40, no. 10, 1 October 1999 (1999-10-01), pages 1644-1651, XP009099772, ISSN: 0161-5505 * the whole document * | 1,14,15 | |
| X | SHIGERU SASE, MORITO MONDEN, HIROSHI OKA, KEIZO DONO, TATSUYA FUKUTA, IEKEDO SHIBATA: "Hepatic blood flow measurements with arterial and portal blood flow mapping in the human liver by means of xenon CT", JOURNAL OF COMPUTER ASSISTED TOMOGRAPHY, vol. 26, no. 2, 2002, pages 243-249, XP009142258, * the whole document * | 1,14,15 | |

TECHNICAL FIELDS SEARCHED (IPC)

-/--

The present search report has been drawn up for all claims

| Place of search | Date of completion of the search | Examiner |
|---|---|---|
| Munich | 9 December 2010 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 17 3819

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WHITE M J ET AL: "Parametric mapping of the hepatic perfusion index with gadolinium-enhanced volumetric MRI", BRITISH JOURNAL OF RADIOLOGY, BRITISH INSTITUTE OF RADIOLOGY, LONDON, GB, vol. 80, no. 950, 1 February 2007 (2007-02-01), pages 113-120, XP009142285, ISSN: 0007-1285, DOI: DOI:10.1259/BJR/36793733 [retrieved on 2006-07-19] * the whole document * | 1,14,15 | |
| X,P | COENEGRACHTS K ET AL: "Perfusion maps of the whole liver based on high temporal and spatial resolution contrast-enhanced MRI (4D THRIVE): Feasibility and initial results in focal liver lesions", EUROPEAN JOURNAL OF RADIOLOGY, ELSEVIER SCIENCE, NL, vol. 74, no. 3, 1 June 2010 (2010-06-01), pages 529-535, XP027180318, ISSN: 0720-048X [retrieved on 2009-04-16] * the whole document * | 1,14,15 | |
| X,P | RAJ A ET AL: "Visualization and segmentation of liver tumors using dynamic contrast MRI", ENGINEERING IN MEDICINE AND BIOLOGY SOCIETY, 2009. EMBC 2009. ANNUAL INTERNATIONAL CONFERENCE OF THE IEEE, IEEE, PISCATAWAY, NJ, USA, 3 September 2009 (2009-09-03), pages 6985-6989, XP031638917, ISBN: 978-1-4244-3296-7 * the whole document * | 1,14,15 | TECHNICAL FIELDS SEARCHED (IPC) |

-/--

The present search report has been drawn up for all claims

| Place of search | Date of completion of the search | Examiner |
|---|---|---|
| Munich | 9 December 2010 | Anscombe, Marcel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                              
& : member of the same patent family, corresponding
document

EPO FORM 1503 03.82 (P04C01)

**Europäisches Patentamt**
**European Patent Office**
**Office européen des brevets**

## EUROPEAN SEARCH REPORT

Application Number

EP 10 17 3819

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2009/112538 A1 (KAROLINSKA INST INNOVATIONS AB [SE]; BLOMQVIST LENNART [SE]; NORDELL A) 17 September 2009 (2009-09-17) * the whole document * | 1,14,15 | |
| X | HWANG EUI-HYO: "99mTc-GSA dynamic SPECT for regional hepatic functional reserve estimation: Assessment of clinical value for hepatic resection", KAKU IGAKU, vol. 36, no. 4, May 1999 (1999-05), pages 323-331, XP009142250, ISSN: 0022-7854 * the whole document * | 1,14,15 | |
| X | ATSUSHI NANASHIMA ET AL: "Relationship Between CT Volumetry and Functional Liver Volume Using Technetium-99m Galactosyl Serum Albumin Scintigraphy in Patients Undergoing Preoperative Portal Vein Embolization Before Major Hepatectomy: A Preliminary Study", DIGESTIVE DISEASES AND SCIENCES, KLUWER ACADEMIC PUBLISHERS-PLENUM PUBLISHERS, NE, vol. 51, no. 7, 1 July 2006 (2006-07-01), pages 1190-1195, XP019394027, ISSN: 1573-2568, DOI: DOI:10.1007/S10620-006-8031-X * the whole document * -/-- | 1,14,15 | TECHNICAL FIELDS SEARCHED (IPC) |

The present search report has been drawn up for all claims

| Place of search | Date of completion of the search | Examiner |
|---|---|---|
| Munich | 9 December 2010 | Anscombe, Marcel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.........................................................................
& : member of the same patent family, corresponding
document

EPO FORM 1503 03.82 (P04C01)

Europäisches
Patentamt

European
Patent Office

Office européen
des brevets

## EUROPEAN SEARCH REPORT

Application Number

EP 10 17 3819

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NORDELL A ET AL: "Liver Function Test Using SVD-Based Deconvolutional Analysis in Gd-EOB-DTPA-Enhanced MRI", INTERNATIONAL SOCIETY FOR MAGNETIC RESONANCE IN MEDICINE. SCIENTIFIC MEETING AND EXHIBITION. PROCEEDINGS, INTERNATIONAL SOCIETY FOR MAGNETIC RESONANCE IN MEDICINE, US, [Online] vol. 14, 6 May 2006 (2006-05-06), page 2204, XP002528515, ISSN: 1524-6965 * the whole document * | 1,14,15 | |
| X | ANDREA LAGHI: "Multidetector CT (64 Slices) of the liver: examination techniques", EUROPEAN RADIOLOGY, SPRINGER, BERLIN, DE, vol. 17, no. 3, 29 September 2006 (2006-09-29), pages 675-683, XP019490357, ISSN: 1432-1084 * the whole document * | 1,14,15 | |
| X | JONAS E ET AL: "Dynamic 99Tcm-HIDA SPET: non-invasive measuring of intrahepatic bile flow. Description of the method and a study in primary sclerosing cholangitis", NUCLEAR MEDICINE COMMUNICATIONS, LIPPINCOTT WILLIAMS AND WILKINS, US, UK, vol. 22, no. 2, 1 February 2001 (2001-02-01), pages 127-134, XP009132310, ISSN: 0143-3636 * the whole document * -/-- | 1,14,15 | TECHNICAL FIELDS SEARCHED (IPC) |

The present search report has been drawn up for all claims

| Place of search | Date of completion of the search | Examiner |
|---|---|---|
| Munich | 9 December 2010 | Anscombe, Marcel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Application Number

EP 10 17 3819

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROBIN MARTIN ET AL.: "Semi-automatic feature delineation in medical images", APVIS '04 PROCEEDINGS OF THE 2004 AUSTRALASIAN SYMPOSIUM ON INFORMATION VISUALISATION, vol. 35, 2004, pages 127-131, XP040025463, ISBN: 1-920682-17-1 * the whole document * ----- | 1,14,15 | |

TECHNICAL FIELDS
SEARCHED (IPC)

The present search report has been drawn up for all claims

| Place of search | Date of completion of the search | Examiner |
|---|---|---|
| Munich | 9 December 2010 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

      .................................................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 10 17 3819

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2010

| Patent document cited in search report | | Publication date | Patent family member(s) | | Publication date |
|---|---|---|---|---|---|
| WO 2007053676 | A2 | 10-05-2007 | CN 101310304 A | | 19-11-2008 |
| | | | EP 1952347 A2 | | 06-08-2008 |
| | | | US 2008103385 A1 | | 01-05-2008 |
| US 2005201601 | A1 | 15-09-2005 | NL 1028550 C2 | | 11-11-2009 |
| | | | NL 1028550 A1 | | 16-09-2005 |
| WO 2009112538 | A1 | 17-09-2009 | AU 2009224632 A1 | | 17-09-2009 |
| | | | CA 2718781 A1 | | 17-09-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090124907 A
- US 20070016016 A
- US 6582370 B
- JP 2003033349 A
- US 7315638 B
- US 20070242069 A
- JP 2009148422 A
- JP 2008050615 A
- US 7046833 B
- US 20080260226 A
- US 20060098256 A
- US 20080044080 A
- US 6973212 B

### Non-patent literature cited in the description

- **S. Saito.** *Journal of Japanese Society of Radiological Technology,* 2008, vol. 51, 30-33
- **Y. Kato.** *Nikkei Medical Online,* 10 July 2009, http://medical.nikkeibp.co.jp/leaf/all/search/cancer/news/20080 2/505562.html
- **H-K. Ryeom et al.** Quantitative Evaluation of Liver Function with MRI Using Gd-EOB-DTPA. *Korean Journal of Radiology,* 2004, vol. 5 (4), 231-239
- **R. Beichel et al.** Liver Segment Approximation in CT Data for Surgical Resection Planning. *Medical Imaging 2004: Image Processing, Proceedings of the SPIE,* 2004, vol. 5370, 1435-1446
- **D. Rueckert et al.** Nonrigid Registration Using Free-Form Deformations: Application to Breast MR Images. *IEEE Transactions on Medical Imaging,* 1999, vol. 18 (8), 712-721
- **Y.Y. Boykov ; M-P. Jolly.** Interactive Graph Cuts for Optimal Boundary & Region Segmentation of Objects in N-D Images. *Proceedings of ''International Conference on Computer Vision,* 2001, vol. I, 105-112